(19) Europäisches Patentamt
European Patent Office
Office européen des brevets

(11) **EP 1 350 431 B1**

(12) **EUROPEAN PATENT SPECIFICATION**

(45) Date of publication and mention
of the grant of the patent:
**25.05.2011 Bulletin 2011/21**

(21) Application number: **02729372.9**

(22) Date of filing: **08.01.2002**

(51) Int Cl.:
*A01N 25/00* (2006.01)   *A01N 43/80* (2006.01)
*G01N 33/15* (2006.01)   *G01N 33/48* (2006.01)
*G06F 17/30* (2006.01)   *C02F 1/50* (2006.01)
*C12N 15/11* (2006.01)   *C12Q 1/04* (2006.01)

(86) International application number:
**PCT/JP2002/000012**

(87) International publication number:
**WO 2002/054865 (18.07.2002 Gazette 2002/29)**

(54) **METHOD OF SELECTING ANTIMICROBIAL AGENT AND METHOD OF USING THE SAME**

METHODE FÜR DIE WAHL EINES ANTOMIKROBIELLEN MITTELS SOWIE IHRE ANWENDUNG

PROCEDE DE SELECTION D'UN AGENT ANTIMICROBIEN ET PROCEDE D'UTILISATION DE CE DERNIER

(84) Designated Contracting States:
**DE ES PT SE**

(30) Priority: **09.01.2001   JP 2001001427
10.07.2001   JP 2001209845
29.11.2001   JP 2001365004**

(43) Date of publication of application:
**08.10.2003 Bulletin 2003/41**

(73) Proprietor: **Kurita Water Industries Ltd.
Shinjuku-ku, Tokyo 160-8383 (JP)**

(72) Inventors:
• **IIZUMI, Taro
Tokyo 160-8383 (JP)**
• **SUZUKI, Hiroyuki
Tokyo 160-8383 (JP)**
• **TASHIRO, Hirohisa
Tokyo 160-8383 (JP)**

(74) Representative: **HOFFMANN EITLE
Patent- und Rechtsanwälte
Arabellastraße 4
81925 München (DE)**

(56) References cited:
**EP-A- 0 822 261        WO-A-97/48822
WO-A-99/34013        JP-A- 4 293 497
JP-A- 7 274 999        JP-A- 11 057 731
JP-A- 2001 004 590**

• **KLAHRE J ET AL: "Monitoring of biofouling in papermill process waters" WATER RESEARCH, ELSEVIER SCIENCE PUBLISHERS, AMSTERDAM, NL, vol. 34, no. 14, 1 October 2000 (2000-10-01), pages 3657-3665, XP004228647 ISSN: 0043-1354**
• **FLEMMING H.-C.: "biofouling in water systems- cases, causes and coutermeasures" APPL. MICROBIOL. BIOTECHNOL., vol. 59, 2002, pages 629-640, XP002310946**
• **KOMA HIROKI ET AL: "Biofouling inhibitors for industrial aqueous systems" HCAPLUS, 14 February 1990 (1990-02-14), XP002231338**

EP 1 350 431 B1

**Description**

FIELD OF THE INVENTION

The present invention relates to a method for analyzing cling substances formed on products of paper manufacturing, in which the microbial biota of microbes that form the cling substances, such as defects and spots, on the product or the dominant microbe therein is identified by making use of base sequence of DNA as a parameter, to a method for inspecting the cause of formation of the cling substances and the location of origin of occurrence of the microbes from the result of such analysis and to a method for controlling occurrence of microbes to enable reduction of occurrence of cling substanses by having resort to the inspected cause of the cling substance formation.

BACKGROUND OF THE INVENTION

It is well recognized that the kinds of microbes appearing in an objective system, to which industrial antimicrobial agents, such as slime control agents and preservatives, are to be applied, are distributed over a very wide range, since such objective system is found in regions very wide not only in view of the microbiological substrate condition but also in view of the environmental condition. For instance, the white water system in paper manufacturing process is operated with white water delivered from a paper machine, which may have varying pH condition ranging from acidic to weakly alkaline pH value and varying temperature condition ranging from about 15 °C in winter season to about 50 °C upon heating. Objective systems to be treated with preservative may be in varying pH condition ranging from acidic to alkaline values together with widely varying temperature conditions ranging from 20 to 70 °C.

Thus, the environmental conditions in the objective system to which the antimicrobial agent is applied are different for each specific system over a wide range and, therefore, the kinds of microbes appearing therein may also be different for each specific system. For selecting the antimicrobial agent to be applied to each specific objective system, it is necessary to select those which are adapted to the microbes and their populations present in the objective system (biota) and are adapted also to the environmental condition therein, since the environmental condition of the objective system has influence also on the effect of antimicrobial agent. If faulty selection of antimicrobial agent is made, not only the expected antimicrobial effect cannot be attained but also useless outflow of considerable amount of antimicrobial agent over natural environment accompanies which may occasionally lead to unexpected destruction of environment.

For dealing with such widely ranging objective systems (application fields), a contrivance for enabling selection of optimum antimicrobial agent for each specific objective system in practice has been proposed, in which a variety of antimicrobial agents exhibiting different properties are provided for use. On growing variety of antimicrobial agents to be provided, however, it has become important to bring about a technique for selecting antimicrobial agents which are most adapted to each specific different condition.

Heretofore, it has, in many cases for applying an antimicrobial agent to an objective system, been practised to determine the antimicrobial agent to be used, in such a manner that a sample of microbes is collected from the objective system, whereupon comparison of effect of antimicrobial agent on the microbes is made for antimicrobial agents of as many sorts as possible, by performing, for example, sterilizing experiments growth inhibiting experiments and bacteria count determination, after the sample has been carried back to the laboratory, and the determination of the antibacterial agent to be used is realized based on the results obtained thereby.

However, it is impossible to carry out the experiments for all the existing antimicrobial agents by the above-mentioned prior technique within a brief period in the point of view of limitation of manpower and time. Thus, there was a problem that the selection of antimicrobial agent had to be realized usually based on the results obtained by experiments carried out with a limited number of antimicrobial agents at limited experimantal concentrations.

Moreover, the prior technique involves a problem that the antimicrobial agents are selected based on the experimantal results for only the microbes which are culturable. Thus, it has been recognized that many microbes which are not able to be cultured isolatedly, namely, so-called non-culturable microbes, are present in the objective system to which antimicrobial agents are applied. In an objective system in which such a microbe not isolately culturable constitutes the dominant species, an antimicrobial agent selected based on the result of experiment involving the operation of culture should not always represent the optimum antimicrobial agent.

As given above, it was the actual matter of fact that the prior technique suffers from a problem that the microbial biota in the objective system may not be made clear by the prior technique and, thus, it is not always clear whether optimum antimicrobial agent has been selected or not, so that selection of antimicrobial agent and application thereof had to be decided by relying largely on experiences.

As to the technique for analyzing microbial biota, there has been practised a classical method of identification in which the microbes are classified based on the morphological/physiological characteristic features, wherein correct results may only difficultly be obtained by a professional skilled in the practice of identification of microbes and, in addition, a long period of time of about one month or more is required for attaining a microbial biota analysis of one single objective system even by a full attendant work by a skilled professional, precluding general application thereof except for a special research purpose. Therefore, it is practically impossible to apply the classical method of identification of microbial biota for routine work of selection of antimicrobial agent.

On the other hand, prompt identifying kits have been brought to the market, which are capable of identifying microbes of a microbial biota within a short term of 7 to 15 days. These identifying kits were, however, developed mainly for a purpose of medical use and, thus, may suffer from a problem that a correct identification of microbes is only dificultly attained, when the kit is used for an objective system to which industrial antimicrobial agent is to be applied. Further, these kits may suffer from a problem that they are applicable only to culturable microbes and erroneous judgements may be derived when used for an objective system in which the dominant microbe can not be cultured isolatedly in a culture medium.

As described above, it is necessary for performing antimicrobial treatment to select an antimicrobial agent suitable for correctly grasping the microbial biota in the objective system within a short period of time and adapted to the microbial biota and environmental condition of the objective system.

As for the techniques for analyzing microbial biota, methods have recently been developed, in which differences in the base sequence of a DNA encoding ribosomal RNA (in the following referred to as rRNA) of microbe, which is referred to hereinafter as rDNA, are utilized, whereby it is now made possible to clearly identify within a brief time microbial biota including non-culturable bacteria not capable of forming colony. However, there has hitherto been known no technical measure of selecting an antimicrobial agent which is at the most adapted to each specific objective system, by combining the information as to the microbial biota identified in the manner as above with a plurality of informations including the efficacy of antimicrobial agent to be used, the nutritive condition of the objective system and the environmental condition.

By the way, a phenomenon has been known, that the antimicrobial effect of an antimicrobial agent may suddenly be changed when one and the same antimicrobial agent is used for a prolonged period of time, due to, for example, a minute variation in the environmental condition or acquirement of a reagent-resistance by a microbe. For this reason, it is necessary to watch periodically whether the antimicrobial agent used does reveal successively its effect as expected or not. For this purpose, there has been practised heretofore to watch the state of staining inside the objective system directly after the shutdown or to perform viable count determination of the objective system by agar plate method to judge the effect.

By the above watching method of prior art, however, it is not able to make clear whether the cause of change in the effect of the antimicrobial agent is due to appearance of a reagent-resistant microbe or due to a simple shortage of amount of the reagent. For this reason, an alternative technique for watching the effect of industrial antimicrobial agent permitting to monitor variation of microbial biota after the application of the antimicrobial agent is required.

In the case where trouble still occurred in spite of use of antimicrobial agent or where a sudden decrease of the effect of antimicrobial agent occurred during a prolonged successive use of one and the same antimicrobial agent, it is required to take necessary step at once to resolve the problem by making clear the cause therefor. In the past, however, there was no technical measure to detect the cause of occurrence of the trouble promptly and assuaredly, except to have resort to a contrivance of responding measure by trial and error.

For such cases as above also, a technical measure for deciding a scientific way of detecting the cause and an adaptive way of treatment permitting to respond even to such urgent cases is requested.

Paper manufacturing process in paper factory comprises dispersing pulp in water to form a suspension, preparing the suspension at a desired fiber size and desired pulp concentration and, then, subjecting the suspension to papermaking after addition of papermaking reagents. The paper manufaturing arrangement is constituted of a pulp raw material preparation system, a paper broke system, a papermaking reagent preparation system, a stock preparation system, a white water circulation system, a white water recovering system and so on. The aqueous liquor separated from the paper machine is called white water which is reused for dispersing pulp to form a suspension, for adjusting the concentration of suspension and for others. White water contains starch and other organic materials added as papermaking chemicals and is maintained usually at a temperature of 30 - 40 ˚C and, thus, is held under a condition preferable for growth of microbes, so that it contains usually microbes at a population of about $10^6$ - $10^7$ per milliliter when no slime control measures are taken.

These microbes proliferate while being held attached on submerged solid walls in white water circulation system of paper machine and may form cling substance called a slime thereon together with non-biological solid matters in white water taken up therein. When such a slime is detached during the operation of paper machine, the paper product will be stained by "spot" or "fish eye", bringing about various troubles, such as deterioration of product quality and even break down of paper.

A means for inhibiting slime formation has now widely been incorporated by adding slime control agent to the white water circulation system, in order to prevent slime troubles, wherefor various slime control agents have been found in the market.

Microbes found in paper manufacturing process courses are brought thereinto from various origins, such as the raw pulp, various papermaking chemicals, industrial water and atmospheric air, and grow and proliferate in various paper manufacturing process courses to form each a microbial biota adapted to each environmental condition. Since these microbes flow along the flow course of paper manufacturing process and will gather in the papermaking raw stock, the white water system contains microbes proliferated in all the paper manufacturing process cources. Among these microbes, those which are adapted to the environmental condition of the white water system and adherent easily onto solid walls to grow and proliferate thereon will constitute main constituent microbes of slime.

From these circumstances, it has been presumed that the microbial biota in the white water system may be complicated, so that slime control agents having wide antimicrobial spectra are apt to be preferred to use. However, slime control agents having wide antimicrobial spectra and higher activities may on the other hand exhibit higher toxicities or higher dangerousnesses in handling, so that there may scarcely be able to get a slime control agent having ideal performances and, hence, use of a plurality of slime control agents in combination has found wide applications.

If the microbial biota in the white water system or in a slime can be analyzed easily and if the site of origin of occurrence of each microbe, namely, where each microbe has mainly proliferated, can be determined, inhibition of slime formation will become possible by a suitable contrivance of a rational way of application of antimicrobial agent, even if the antimicrobial spectrum thereof is not ideally wide. Thus, it has to be assumed that a stable slime control effect can be attained, when a slime control agent effective for the dominant microbe in the white water system is added to the white water system and a further slime control agent effective for a microbe exhibiting resistance against the aforementioned antimicrobial agent is added to the site of origin of occurrence of such microbe.

Such an idea has been proposed hitherto and a method has been attempted, in which a slime control agent is added also to the site at which proliferation of microbes is particularly intensive, such as the broke chest. In this prior method, the manner of determination of the site of origin of occurrence of microbes consists in a practice of collecting a sample from each process course of paper manufacture and performing viable count determination by, for example, agar plate method, wherein the site where the viable count is higher is determined as the site of origin of occurrence of microbes.

In the above prior method, it is not able to judge whether the existing microbes contain a proliferated microbe resistant against the slime control agent (white water treating agent) presently on use or are all susceptible thereto, since the prior art viable count measurement does not permit identification of each kind of microbe. Therefore, it has been necessary to select the slime control agent to be added to the site of origin of occurrence of microbes (origin treating agent) by collecting a sample again from each of the sites where the viable counts were higher and performing antimicrobial experiments using various slime control agents.

If a microbe resistant against the white water treating agent constitutes the dominant microbe species in the microbial biota in the origin of occurrence in which microbes grow, the above prior art method may permit to attain selection of pertinent origin treating agent so long as the bothersome operation can be endured. However, when the circumstances are such that the dominant microbe is susceptible to the white water treating agent but a kind of microbe resistant to the treating agent also grows concurrently, the prior art method may difficultly permit to determine pertinent origin treating agent or even be impossible to determine.

In the process courses of paper manufacturing, reuse of white water has progressed and has reached the state that it is repeatedly used in circulation in every process course in order to spare consumption of water. In a paper manufacturing factory where reuse of white water has progressed, the viable count in various process liquors becomes not different from that in the white water, so that determination of site of origin of microbe occurrence has become impossible by viable count determination. On the other hand, occurrence of slime troubles is enhanced in general in systems where reuse of white water has progressed, a more rational method for inhibiting slime formation is highly requested.

On the other hand, the most important problem in the quality control in a paper manufacturing factory comprising the above-mentioned paper manufacturing process courses resides in that any kind of alien substance adheres onto the paper upon papermaking and causes occurrence of defective portion called a "defect" (also called "hole", "spot" or "fish eye") on the product. Such defect will not only deteriorate the product quality considerably but also cause various troubles upon printing on the paper. In addition, such a defect may cause break down of paper, rendering operation of paper machine difficult and decreasing the productivity considerably.

It has been known that the substance causing the defect includes a slime (constituted of microbes and mucous substances produced therefrom), pitch, scale, sizing agent, contaminant inclusion and composite complex of them. In papermaking process course in paper manufacturing, formation of slime originated from microbes has attracted great attention, since this process course is held under a condition favorable for the growth of microbes, so that a slime control treatment is incorporated in general for inhibiting formation thereof.

Whether the defect formed on the product is derived from microbial slime or not can be judged by, for example, observation under a microscope or chemical analysis, such as ninhydrin reaction. If it is judged that the defect is derived from microbial slime, the manner of slime control treatment must be improved to prevent occurence of defect.

However, it may be difficult to identify each microbe by separating and culturing the microbe from the defective portion of paper product and to determine the existence proportion of microbes therein, namely, the microbial biota, and the kind of the dominant microbe, since paper product has been subjected to high temperature drying. Therefore, it is impossible to identify what kind of microbe causes the defect (in the following, sometimes referred to as causal microbe) and which one among the paper machine, water systems and raw material system has to be judged as the origin of occurrence of the causal microbe.

In the past, an example has been reported in which a part of microbes forming stout spores was separated from paper (Vaisanen et al; Journal of Appl. Bacteriology, 71, pp 130 - 133, 1991), in which also identification of causal microbe for the defect and determination of the original site of occurrence of such causal microbe are prohibited, since almost all the microbes are extinct.

Therefore, it has been impossible to realize a scientific and rational response, such as identification of causal microbe and determination of original site of occurrence thereof with subsequent microbe control treatment by a method which is at the most adapted to the microbe. In these circumstances, the counter measure presently used has been only based on a "symptomatic treatment", such as increasing the amount of the slime control agent at the paper machine by an empirical and circumstance-responded judgement or anticeptically treating a site where viable count is relatively high, such as chests or the like, so that a reliable reduction of occurrence of defect caused by microbes may difficultly be attained.

An object of the present invention is to propose a method for inspecting the causal basis of occurrence of cling substance on products of manufacture in paper manufacturing process, which permits determination of site of origin of occurrence of the microbe that caused the defect on paper products in a simple and reliable manner.

A still further object of the present invention is to propose a method for controlling microbes in paper manufacturing process, which permits determination of the site of origin of occurrence of the microbe that caused the defect on paper products in a simple and reliable manner, whereby reduction of occurrence of defect on paper products can be attained in a simple and reliable manner.

## BRIEF DESCRIPTION OF THE DRAWINGS

Fig. 1 is a flow diagram of the apparatus for paper manufacturing, to which the process for effecting antimicrobial treatment is to be applied, shown in one mode of implementation of the invention.

Fig. 2 is a flow diagram showing an embodiment of the method for analyzing microbial biota in the process for effecting antimicrobial treatment and the method for selecting the antimicrobial agent , shown in one mode of implementation of the invention.

Fig. 3 is a flow diagram of a paper manufacturing apparatus to which the method for slime control is to be applied, shown in one mode of implementation of the invention.

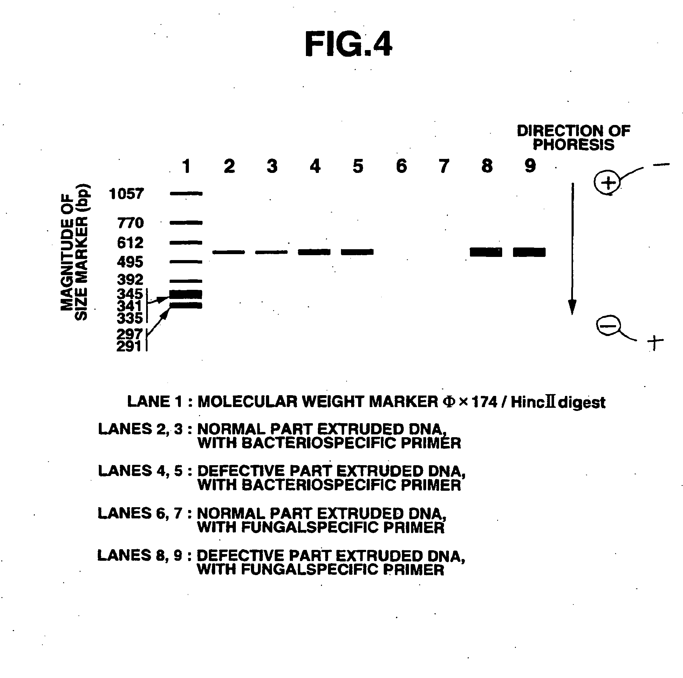

Fig. 4 is a diagram showing the result of electrophoresis experiment performed in Example 10.

Fig. 5 gives graphs each showing the fragment patterns of DNA analyzed in Example 11 obtained by TRFLP method. In all the graphs, ordinate refers to fluorescence intencity and abscissa refers to the length of TRF (Terminal Restriction Fragment) in bases.

## DISCLOSURE OF THE INVENTION

The microbial biota of the objective system to which an antimicrobial agent is to be applied cannot be analyzed by conventional classical method for identifying the microbes and, therefore, selection of an antimicrobial agent in accordance with each specific microbial biota in the objective system has, in fact, not been possible. In these circumstances, the present inventors had paid their special attention to the fact that analysis of microbial biota by making use of base sequence of DNA can be realized within a brief time together with an advantage of permission of analysis of even a microbe that cannot be isolated by culture in culture medium. Based on such idea, the inventors reached completion in a reference aspect of a process for effecting antimicrobial treatment which permits to select an adaptive antimicrobial agent in accordance with each specific microbial biota, by combining such idea organically with the results of experiments performed for various microbes and with the practical data of experiences of treatment performed on various objective systems using antimicrobial agents. Further, the inventors have completed a method in a reference aspect in which the effect of antimicrobial treatment is monitored by watching variation in the microbial biota in an objective system after application of antimicrobial agent to the system, whereupon the antimicrobial agent used is changed, if necessary, to other one.

Furthermore, the present inventors recognized that the analysis of microbial biota by making use of base sequence of DNA is far superior as compared with the conventional classical technique in the points of accuracy and promptness and is effective for comparing microbial biotas and for determining the site of origin of occurrence of microbe in paper manufacturing process courses, which has, with further sound researches, led to the completion of the present invention.

Moreover, the present inventors recognized that it is possible to attain analysis of the kinds of microbes constituting cling substances on paper products, their names, constitutional proportion, dominant microbe and so on, by extracting from defective portion on the paper product each DNA originated from each microbe and utilizing the genetic informations of the DNA, wherefrom the present invention has been completed.

Thus, the present invention resides in

1. A method for inspecting the location of the origin of occurrence of the microbes that causes cling substances on products of manufacture in paper manufacturing process, comprising

a microbe analyzing step of the products comprising extracting from the cling substance adhering on the product of manufacture the DNA originated from each microbe, and performing analysis of the microbial biota or the dominant microbe of the cling substance by making use of the resulting base sequence of DNA as analysis parameter,

a microbe analyzing step of the slimes comprising collecting slimes at at least two locations in the paper manufacturing process selected from the group consisting of pulp raw material preparation system, broke system, paper manufacturing chemical preparation system, stock preparation system, white water circulation system and white water recovering system, extracting from the collected slimes the DNA originated from each microbe, and performing analysis of the microbial biota or the dominant microbe of the slimes by making use of the resulting base sequence of DNA as analysis parameter, and

a determination step comprising comparing the microbial biota of the dominant microbe in the cling substance with the microbial biota or the dominant microbe in the slimes collected at said at least two locations in the paper manufacturing process courses, identifying the slime that caused the cling substance by judging the slime of which the microbial biota or the dominant microbe that is in accord with or close to that in the cling substance as the causal slime, and determining the location of occurrence of the slime that caused the cling substance by judging the location where the identified slime has been collected as the location of occurrence of the causal slime.

2. A method for controlling microbes that causes cling substances on products in paper manufacturing process courses, comprising

a microbe analyzing step of the products comprising extracting from the cling substance adhering on the product of manufacture the DNA originated from each microbe, and performing analysis of the microbial biota or the dominant microbe of the cling substance by making use of the resulting base sequence of DNA as analysis parameter,

a microbe analyzing step of the slimes comprising collecting slimes at at least two locations in the paper manufacturing process selected from the group consisting of pulp raw material preparation system, broke system, paper manufacturing chemical preparation system, stock preparation system, whitewater circulation system and whitewater recovering system, extracting from the collected slimes the DNA originated from each microbe, and performing analysis of the microbial biota or the dominant microbe of the slimes by making use of the resulting base sequence of DNA as analysis parameter,

a determination step comprising comparing the microbial biota or the dominant microbe in the cling substance with the microbial biota or the dominant microbe in the slimes collected at said at least two locations in the paper manufacturing process courses, identifying the slime that caused the cling substance by judging the slime of which the microbial biota or the dominant microbe that is in accord with or close to that in the cling substance as the causal slime, and determining the location of occurrence of the slime that caused the cling substance by judging the location where the identified slime has been collected as the location of occurrence of the causal slime, and

a controlling step comprising performing an antimicrobial/antiseptic treatment at the so-determined location of occurrence of the slime.

3. The method as claimed in item 1 or 2, wherein the DNA to be used for analyzing the microbial biota or the dominant microbe is that which codes ribosomal RNA.

In the context of this specification, "microbe" encompasses bacteria, yeast, fungi (mold), algae and archaebacteria etc.

The "antimicrobial agent" does mean a reagent having biocidal effect and/or growth inhibiting effect on the above microbes and includes in general those which are called antibacterial agent, bactricide, bacteriostatic agent, growth inhibitor, slime control agent and preservative.

The word "antimicrobe" does mean a function of making such microbe extinct and/or of inhibiting growth thereof.

The word "microbial biota" is used in the meaning of representing each kind of the microbes in an objective system or in a sample, proportion of their constitution and contents thereof (population or proportion of microbe amount), while it may also be used occasionally in the meaning of representing kind of dominant microbe, proportion of constitution thereof and content thereof or representing kind of specific microbes and their proportion and content.

The terms "result of experience of practical treatment" and "effect of treatment" mean both the effect upon the application of antimicrobial agent to an objective system. This effect may be expressed also by the difference in the amount of growth of slime (permissible also by the difference in the amount of the microbe or in the thickness of the slime), for the case of, for example, using a slime control agent in paper manufacturing process courses. Expression by the period of time till a definite amount of growth is reached may also be permitted. It may include representation by the degree of intrinsic trouble caused by the slime formation in the objective system.

The "white water treating agent" means a slime control agent to be used in the white water circulation system. The "origin treating agent" means a slime control agent to be used by adding it to the site of origin of occurrence of the reagent-resistant microbe.

The word "cling substance" means a contaminant matter occurring on a paper product called defect (also called hole, spot or fish eye).

The word "data base for search for antimicrobial agent" is used in the same meaning as "data base for search for treating agent".

O Method for selecting antimicrobial agent (reference aspect)

The method for selecting antimicrobial agent comprises
a microbe analyzing step in which microbes in the microbial biota of a sample are analyzed based on base sequence of DNA and
an antimicrobial agent selecting step comprising performing search in a data base storing data for industrial antimicrobial agents applicable to the analyzed microbes recited at effective concentrations, picking up industrial antimicrobial agents effective for the microbial biota, dominant microbe or specific microbe(s) in the sample analyzed in the above microbe analyzing step and selecting among the picked up ones one or more industrial antimicrobial agent.

Such a method for selecting antimicrobial agent can be utilezed for selecting an antimicrobial agent (slime control agent) in, for example, antimicrobial treatment as mentioned above, monitoring antimicrobial effect, inhibition of slime formation in paper manufacturing process courses and inhibition of occurrence of microbes in paper manufacturing process.

In the method for selecting antimicrobial agent , microbial biota is analyzed in the microbe analyzing step based on base sequence of DNA, wherein known techniques can be utilized for the analysis of microbial biota without any limitation. In the analysis of microbial biota, determination (identification) by nomenclature for each microbe may not always be necessary, but instead, may assign therefor private address number permitting distinction from other microbes (in the following referred to as microbe number) for use in the place of academic nomenclature. For instances, when a microbe having high homology with the analyzed one is not retrieved from the data base for search for microbes, it is possible to assign therefor a microbe number originally to distinguish from other microbes. However, it may be favorable to identify microbes by each nomenclature based on base sequence, since use of name of each microbe (by nomenclature) may be favorable in putting information of the microbial biota to public.

For identifying (deciding) a microbe, it is able to have resort to, for example, a data base of phylogenetic classification of microbes (data base for search for microbes) in which relationships between the base sequences of DNA and microbes are stored. By search in this data base using a computer, such as personal computer, a microbe to be dealt with can be identified. Access to a definite data base for search for microbes, which is described later, can be realized via internet, so that search for data can be attained promptly. In the data base for search for microbes as described later, data for base sequenses of various DNA that encode ribosomal RNA (rRNA), denoted as rDNA, are stored, so that base sequence of each rDNA isolated from the sample can be utilized for identifying the microbe to be dealt with. While rDNA sequences are classified into several classes (such as 16S DNA, 18S rDNA and so on) in accordance with the kind of microbe and the size of subunit, any sequence may be permitted to use. It is permitted to utilize for the technique according to the preset invention not only rDNA sequences but also other DNA regions and genes, such as the spacer sequences of rDNA, gyrE and so on. Therefore, whatever data bases for search for microbes may be permitted to utilize, wherein utilization of data bases in a form integrated into one unit may also be permitted. It is also permitted to utilize a plurality of data bases concurrently.

For the data base for search for microbes, there may be enumerated, for example, public DNA data bases, such as GenBank, EMBL and DDBJ, as well as Ribosomal Database Project installed in the University of Michigan. Operation of search can be realized in a brief time efficiently by existing programs, such as FASTA and BLAST and the like. It is also possible to search in a commercial data base, such as MicroSeq 16S rDNA Sequence Database (trademark, of PE Biosystems Japan, Ltd.), using a commercially available software, such as MicroSeq Analysis Software (trademark, of PE Biosystems Japan, Ltd.). It is also permitted to construct a data base for search for microbes privately. For instance, a data base may be constructed from data for base sequences so as to permit to attain search for the name of microbe corresponding thereto or for the microbe number permissible of distinguishing the microbe using, for example, known data base software, which may be used concurrently with other data base for search for microbe.

From a sample collected from the objective system, through isolation of colony of a microbe, the base sequence of DNA corresponding to the isolated strain is determined, whereupon the obtained result is compared with the data from the data base, whereby the microbe can be identified. While rDNA can be isolated and amplified by classical cloning techniques using a host/vector system of, for example, E. coli or so on, it is simple and convenient to perform amplification by means of an in vitro amplification of DNA, such as PCR (Polymerase Chain Reaction) technique.

It is also possible to identify microbes by extracting DNA of the microbes in mixture without isolating each microbe by culture, effecting amplification of only rDNA of each microbe by in vitro amplification of DNA, such as the PCR technique, using a primer constituted of a base sequence having high degree of commonness to most microbes to be dealt with, isolating each rDNA originated from each individual microbe by, for example, gel electrophoresis, and comparing the obtained result with the data from the data base.

In the case of identifying a plurality of microbes, a rough distinction between them may be attained by means of the technique by RFLP (Restriction Fragment Length Polymorphism; Moyer et al, Applied and Environmental Microbiology, 62, 2501 - 2507 (1996)) before determination of base sequences. Namely, rDNA sequences of the microbes are subjected to complete digestion using various restriction enzymes and are then subjected to separative electrophoresis on polyacrylamide gel or agarose gel, whereupon they can be distinguished based on the difference in the pattern after staining the DNA.

In a technique in which each microbe is detected by isolation of colony among a group of microbes, a nonculturable microbe, if present, can not be detected. Even for microbes which can be subjected to isolate culture in culture medium, there is no assurance that all the culturable microbes are detected with only one single culture medium or culture condition. On the other hand, in a technique in which each microbe is detected by extracting microbial DNA from mixture of microbes without isolating each individual microbe, there are differences between microbes in the extraction rate, in the number of copies of corresponding genes, in the amplification rate upon PCR and in the susceptibity which is different among the detected group of microbes by the manner of selection of the primer, though it reveals an advantage that even microbes not permissible of being subjected to isolate culture in culture medium can be detected. Therefore, this technique may be incorporated solely or in an adequate combination with other technique, insofar as the characteristic nature of this technique given above and the characteristic features of microbes found in the objective system has been grasped.

For isolating rDNA of individual microbe from rDNA mixture, electrophoresis is used in general, for which several techniques have been proposed. They are, for example, DGGE (Denatured Gradient Gel Electrophoresis; Muyzer et al, Applied and Environmental Microbiology, 59, 695 - 700 (1993)); TGGE (Temperature Gradient Gel Electrophoresis; Eichner et al, Applied and Environmental Microbiology, 65, 102 - 109 (1999)); SSCP (Single Strand Conformational Polymorphism; Schwieger et al, Applied and Environmental Microbiology, 64, 4870 - 4876 (1998)); TRFLP (Terminal Restriction Fragment Length Polymorphism; Liu et al, Applied and Environmental Microbiology, 63, 4516 - 4522 (1997)) and the random cloning (Dunbar et al, Applied and Environmental Microbiology, 65, 1662 - 1669 (1999)). The method for selecting antimicrobial agent according to the present invention is not limited to the use of these techniques.

Further, it is possible according to each special purpose to use other techniques, such as real time PCR for detecting a specific microbe in a mixed microbe system (Wittwer et al, BioTechnique, 22, 130 - 138 (1997)); FISH (Fluorescence In Situ Hybridization; Amman et al, Applied and Environmental Microbiology, 58, 614 - 623 (1992)) and classical hybridization techniques (for example, William et al, Microbiology, 141, 2793 - 2800 (1995)), for the method for selecting antimicrobial agent according to the present invention. Also for the restriction enzymes and the primers to be employed for these known techniques, for which various ones have been known, volunary known ones may be used.

From isolated and amplidied rDNA, the base sequence thereof can be directly determined by experimental procedures. For such a series of procedures, reagent kits found in the market, such as for example, AutoRead Sequencing Kit (trademark, of Amersham Pharmacia Biotech Corp.) and MicroSeq 500 16S rDNA Kit (trademark, of PE Biosystems Japan, Ltd.) may be used. It is of course possible to carry out procedures by having resort to such a technique as the dideoxy method, by preparing privately reagent, such as DNA polymerase or so on. For the analysis apparatus, a commercially available base sequence analyzing apparatus, such as for example, ALFexpress II DNA Analysis System (trademark, of Amersham Pharmacia Biotech Corp.) or ABI PRISM 310 (trademark, of PE Biosystems Japan, Ltd.) may be employed. It is of course possible to utilize polyacrylamide gel electrophoresis, in which the band positions (sequencing ladder) can be determined by, for example, autoradiography.

For a sample of rDNA mixture, it is permissible to perform in such a manner, that the bands resulting from elecrophoresis are stained and each band is cut out together with the gel, whereupon the so cut out gel is treated so as to extract the DNA followed by purification, before being subjected to PCR again, in order to use the amplified DNA for determining the base sequence of the DNA.

For carrying out search in existing data bases for search for microbes, methods have been put to the public, which can be used within the range assigned for each data base, to permit to avail of.

It can be judged by comparing the detected base sequence of the DNA of a microbe found in the sample with each DNA base sequence registered in the data base, that the microbe found in the data base having a base sequence of DNA which has higher most homology with that found in the sample is the microbe of most close relation thereto.

Between identical microbes, 100 % homology has to be recognized in the base sequence of the gene selected as the parameter, except for some exceptional cases (for example, polymorphism in a plurality of copies on a genome). Between closely related microbes of identical species of identical genus, a homology close to 100 % is recognized, though some difference may be present for each specific kind of the gene. For example, for 16S rDNA, a homology of about 98 % or higher is recognized. Therefore, if a homology of 98 % or higher, preferably 99 % or higher, is recognized on comparison of corresponding base sequence between microbes, they can be judged in general as belonging to identical species of identical genus. If the homology is less than 98 %, they are not judged as belonging to identical species of identical genus but, instead, may be designated by a private microbe number to use it in the place of the microbe name.

The constitional proportions of the identified microbes in the biota can be determined, for example, from proportions of the colonies, proportion of each DNA and so on. In a method in which each colony is isolated to inspect, the constitutioal proportions of these microbes can be calculated by selecting a statistically reliable number of colonies at random and performing identification of microbe for all the selected colonies. In a method in which the DNA for each microbe is directly analyzed from a microbe mixture, it is assumed that the constitutional proportions of microbes may be grasped from the ratio of the observed strength of each DNA band relative to the integrated strength of all the DNA bands, since the strength of a DNA band observed in, for example, the method of DGGE or TGGE, is understood to reflect the population of each microbe. For determining the band strength, a commercially available densitometer or a scanner-cooperating image analysis device {such as for example, ImageMaster (trademark, made by Amersham Pharmacia Biotech Corp.) and others} may be employed. Also, it may be possible to use a DNA primer bound with a fluorescent reagent or, in the case where a fluorescent substance is used for staining the bands after the electrophoresis, it is possible to observe the strength of the fluorescence directly using a fluorescence image analyzer, for example, Fluor-Imager (trademark, Amersham Pharmacia Biotech Corp.). Further, by FISH method, the constitutional proportion of microbes can be obtained by observing count of stained microbe cells for each kind of microbe directly under microscope and calculating the proportion thereof relative to the entire microbe counts.

The absolute amount of microbe individuals per a unit volume or unit weight of culture medium can be determined by a known method, for example, counting the number of colonies on a plate culture medium; a method by MPN (Most Probable Number) or a method of counting individual microbe cells directly under microscope after having been stained with a fluorescent substance, such as DAPI (4,6-diamino-2-phenylindole), acrydine orange or CFDA (carboxyfluorescein diacetate). It is possible to calculate the content of each specific microbe in the medium by multiplying the absolute number by the constitutional proportion of the microbe. In the case of real time PCR method or a classical hybridization method, the starting number of microbe cells or the starting amount of DNA in the tested sample can be presumed using a specific microbe or a specific DNA of known concentration as a reference object and subjecting this reference object to a reaction under the same condition as that for the sample.

In the method for selecting antimicrobial agent , the data base to be used in the antimicrobial agent selecting step (in the following referred to sometimes as data base for search for antimicrobial agent) may be of any type, so long as it stores at least data for microbes and for effective concentrations of various antimicrobial agents for each of these microbes and permits search and picking up of antimicrobial agents of which effective concentrations are recorded, by using, as the search key, a microbe. For the search key, there may be employed, for example, the microbe name, the accession number and the microbe number, detected from data base for search for antimicrobial agent. The microbe may be represented by the scientific name (for example, Escherichia coli HB101 and the like) or by the accession number thereof. It may be permissible to use a privately designated microbe number for such a microbe or a DNA, which is not permitted to identify by scientific name. The search key can be inputted by an inputting device, such as keyboard or the like. The data for effective concentration may be any of those obtained from culture experiment for each microbe, those obtained from experiences in practical treatments or those obtained from existing literatures.

The fact that an effective concentration is recorded does mean that the antimicrobial agent has an antimicrobial effect on the microbe recited there at the recited concentration. Here, it may be permitted that a plurality of concentrations of an antimicrobial agent are recited to indicate presence or absence of antimicrobial effect or to indicate the degree of effectiveness at such concentrations. In such a case, picking up of antimicrobial agent is made at the recited effective concentration of the antimicrobial agent.

For the data base for search for antimicrobial agent, such one may favorably be used which permits addition and accumulation of data as to, for example, the microbe name and the effective concentration of antimicrobial agent therefor, upon each occasion of appearance of unregistered new microbe or new base sequence.

For the data base for search for antimicrobial agent, preference is given also for such one, in which data for effective concentrations of antimicrobial agents for various microbes under each environmental condition having influence on the growth of the microbe are stored and which permits to pick up antimicrobial agents among those given under designated environmental conditions. For concrete examples of environmental condition, there may be enumerated pH, temperature, each specific objective system and the identification class thereof, each specific nutrient substratum and concentration thereof and materials affecting the antimicrobial function, such as reducing agent etc., as well as their concentrations. As to each specific objective system and its identification class mentioned above, there may be recited for papermaking process course, the papermaking technique employed, conditions for papermaking, kind of paper product, the kinds and application concentrations of various internal additives, such as for example, sizing agent, retention aid and paper strengthening aid, and the kind of antimicrobial agent that is used in the past. For cooling water systems, there may be recited, for example, the source of make up water, quaity thereof, gaseous components from atmospheric air contained therein, residence time of cooling water and others including reagents employed. For the objective system for preservative treatment, there may be recited, for example, the objective substratum and the treating period required. By the way, for the case where the effective concentrations of antimicrobial agents recorded in the data base are those obtained by culture experiment, the above-mentioned conditions, such as pH and temperature, belong to culture condition and, for the case where the data are obtained from experiences of practical treatment, the above conditions of pH, temperature and so on fall under the environmental condition of the objective system.

For pH as an environmental condition, it is favorable that effective concentrations at a plurality of pH values are recorded in the data base. For example, the data may preferably be recorded at pH of 5, 6, 7 .... Also as for temperature and other conditions, it is favorable that effective concentrations at a plurality of conditions are recorded in the data base, as in the case of pH. When the data as to the effective concentration are recorded for each environmental condition as above, it is possible to search effective antimicrobial agents using a condition of, for example, pH value of 6 and a temperature of 20 ˚C, as the search key.

For the data base for search for antimicrobial agent, preference is given further for such one, which permits to calculate the difference in the amount of each microbe in the microbial biota before and after the use of antimicrobial agent and to record the result of calculation, when the results of analyses of the microbial biota as well as the results of practical treatment before and after the use of antimicrobial agent are inputted thereinto, permitting thus addition, accumulation and revision of data by performing judgement from the result of calculation as to whether or not the concentration of the antimicrobial agent used was effective for the microbes. By using such a data base for search for antimicrobial agent, a more pertinent antimicrobial agent can be selected.

For the data base for search for antimicrobial agent, preference is given furthermore to such one, which calculates the difference in the amount of each microbe in the microbial biota before and after the use of antimicrobial agent on inputting the analysis results of microbial biota as well as the concentrations of the antimicrobial agent used before and after the use of the antimicrobial agent and judges then, based on the result of calculation, as to whether or not the concentration of the antimicrobial agent used was effective for the existing microbes, followed by comparison of the result of judgement made based on the experiences of practical treatment with the result of judgement made based on culture experiment carried out for the culturable microbes contained in the objective system (i.e. the collected sample) to judge that the effectiveness of the antimicrobial agent in the practical treatment was far greater, within proper range or far lower, as compared with that in the culture experiment, so as to permit addition, accumulation and revision of data. By using such a data base as above, it is made possible to assess the effectiveness of the antimicrobial agent used under actual environmental condition accurately so as to permit to select more pertinent antimicrobial agent.

The data base for search for antimicrobial agent may preferably be such that data for the charactristic properties and antimicrobial effectiveness of antimicrobial agents recited in literaturs etc. as well as for the prices of antimicrobial agents have been recorded therein. The data base may further preferably be capable of addition and accumulation of new data as to, for example, experiences of practical treatments, test results and experimental results.

The data base for search for antimicrobial agent storing more data as given above permits to realize selection of more pertinent antimicrobial agent.

Since the antimicrobial spectrum of an antimicrobial agent for microbes not permitting isolated culture in culture medium cannot be observed by laboratory culture experiment using pure culture of bacterium strain, it is necessary for the information of, for example, effective concentrations of antimicrobial agent for such microbes, to accumulate many data for experiences of practical treatments.

For instance, a practical measure as given below may be helpful: A format for a data base for search for antimicrobial agent may preliminarily be prepared in such a way that the results of analyses of the microbial biota before and after application of an antimicrobial agent as well as the effect of the antimicrobial treatment attained thereby, concentration of the antimicrobial agent maintained in the system (replaceable by the maintained concentration obtained by calculation from the application concentration) and other environmental conditions of the objective system having influence on the growth of microbes and on the antimicrobial effect of the antimicrobial agent are additionally inputted and recorded one by one in the data base upon application of said antimicrobial agent to the system. A column is installed in the data base for search for antimicrobial agent having the obove format, which column is capable of storing, as the result of calculation, the difference in the microbial biota of the system before and after the application of the antimicrobial agent assuming that the microbe(s) present before the application of the antimicrobial agent but disappeared after the application of the antimicrobial agent have been exterminated by the antimicrobial agent applied. By incorporating such a technical measure, it is able to judge that the microbe(s) disappeared after the application of the antimicrobial agent were susceptible to the natimicrobial agent applied at the concentration maintained in the system and, on the other hand, that the microbes showing unchanged or increased population were non-susceptible or resistant to the antimicrobial agent applied at the concentration mantained in the system.

For assessing the propriety of the so-evaluated effectiveness of antimicrobial agent for a series of microbes, there may be employed a method as given below:

The antimicrobial activity determined by a laboratory experiment of a specific culturable microbe found in a sample collected from the objective system is called out from the data base, since such sample contains usually at least one or two or more culturable microbes, in order to judge, by collating the antimicrobial activity obtained by laboratory experiment with the above evaluated effectiveness of the antimicrobial agent, whether or not the said microbe was held in a susceptible range at the concentration of the antimicrobial agent maintained in the objective system, wherein it is judged that the data for the antimicrobial activity determined by calculation in the above application example for a series of microbes are within a proper range when no contradiction is found within a tolerable limit (which may be settled voluntarily) as compared with the result of the laboratory experiment. A column is provided in the data base for recording result of judgement and the above judgement result may be entered in this column. If any data of laboratory experiment is not available, a data from new laboratory experiment using the microbe isolated here may be added in the data base.

When a substance deteriorating the activity of antimicrobial agent, such as a reducing substance, is contained in the objective system to be treated, the antimicrobial effect dertermined in the manner as above should be lower as compared with the antimicrobial effect determined by the laboratory experiment. Thus, the antimicrobial effect should have been affected by contaminant substance present in the system and the data therefor should be an underrated one. Therefore, for the so-obtained series of underrated data, designation of "underrated" may be labelled on the above-mentioned column. When, on the other hand, the rest of the antimicrobial agent used in the forgoing process course is carried over into the objective system, the activity of the antimicrobeial agent used may be overrated. For such a case, a designation of "overrated" may be labelled on the column.

When a format for the data base for search for antimicrobial agent having a writing column as given above is laied down and many data from application examples are accumulated therein, it may be made possible to select an effective antimicrobial agent from a group of data in the proper range, when an antimicrobial agent effective for certain microbes not permitted to culture isolately in culture medium has to be searched.

When, in the case where an antimicrobial agent to be used to apply to an objective system is newly selected, a substance deteriorating or emphasizing antimicrobial activity of such antimicrobial agent is present concurrently in the objective system, the group of data which were judged as "underrated" or "overrated" can constitute an important element for selecting antimicrobial agent, so that it is preferable to store them in the data base without being discarded.

For the computer software for constructing the data base for search for antimicrobial agent to be used in the method for selecting antimicrobial agent , commercially available softwares, such as Micrisoft Exel (trademark) or Microsoft Access (trademark) of Microsoft Corp., though there is no special restriction and any one may be employed, so long as it suffices the purpose. As to the function for permitting search for the contemplated items, for example, activities of antimicrobial agents and environmental conditions, from the corresponding scientific name of a microbe or the accession number, such function is intrinsically provided in such a software and can be availed of. For a software lacking in such function, addition may be permitted in combination with a known technique.

By selecting antimicrobial agent in the manner as given above, an antimicrobial agent which is at the most adapted to the objective system to be treated can easily and accurately be selected within a brief time.

○ Process for effecting antimicrobial treatment (reference aspect)

The process for effecting antimicrobial treatment consists in a technique comprising, instead of using the empirical practice for selecting antimicrobial agent depending on a prior technique, such as for example, sterilizing test, growth inhibiting test or microbial cell counting by agar plate, the steps of analyzing the microbial biota of the objective system based on base sequence of DNA, collating the analyzed microbial biota with a data base capable of search for antimicrobial agent, selecting an antimicrobial agent which is at the most adapted, to avail of it.

The objective system to which the process for effecting antimicrobial treatment is applicable is not specifically limited, so long as it deals with a system treated with antimicrobial agent(s). There may be recited, for example, systems in which one or more industrial slime control agents are used, such as paper manufacturing process courses, cooling water system and ultrapure water production system; and systems, such as starch slurry for paper manufacturing, paste liquor and water-soluble cutting oil for metal machining in which preservative is used.

In the process for effecting antimicrobial treatment a sample is collected first from the objective system. It is assumed that a plurality of kinds of microbe may be present usually in such a sample. Then, the microbial biota of the collected sample is analyzed based on base sequence of DNA, whereupon pertinent antimicrobial agents are selected from the data base. For the analysis of the microbial biota and for the selection of antimicrobial agents, the procedures employed in the microbe analysing step and in the antimicrobial agent selecting step described in the above method for selecting antimicrobial agent can be utilized as such.

The process for effecting antimicrobial treatment comprises, collecting first a sample containing microbes, such as slime, white water, circulating cooling water and objective material for antiseptic treatment, from the objective system and performing analysis of the microbial biota of the collected sample based on base sequence of DNA, in order to identify each microbe constituting the microbial biota of the collected' sample by referring to the microbe name or to the accession number (corresponding to the microbe name). Usually, the series of the above examination operations may be completed within a period of 2 - 7 days.

Then, search in the data base for search for antimicrobial agent is carried out using, as the search key, the microbe identified by the above analysis, whereby antimicrobial agents are searched and picked up. Here, the antimicrobial agents to be searched can be limited into more narrow extent when experimental condition etc are inputted. The result of picking up of the antimicrobial agent may be outputted on an output device, such as a display. If only one antimicrobial agent was picked up, this antimicrobial agent will constitute the selected one and, if a plurality of antimicrobial agents were picked up, selection is made among them. The selection of the antimicrobial agent to be served for actual application among the picked up antimicrobial agents may be carried out in any arbitrary way, for example, by human visual choice, by automatic choice by computer or so on. Concrete manner of selection may include, for example, the following cases:

1) An antimicrobial agent revealing a minimum effective concentration which is at the most low for all the microbes or for the dominant microbe is selected.

2) An antimicrobial agent which is at the most effective for a preliminarily designated specific microbe is selcted.

3) Selection is effected in such a way that a certain microbe which is resistant against the antimicrobial agent shall be neglected for its presence, when the occupation proportion thereof is not higher than an arbitrarily settled limit, but the selection shall be made only with respect to the antimicrobial effect for the microbe, when the occupation proportion exceeds over the settled limit.

4) An antimicrobial agent, with which the value

$$[(\texttt{minimum effective concentration}) \times (\texttt{price})]$$

is the lowest for the dominant microbe or for a certain specific microbe, is selected. In this case, selection can be attained taking into account of treating cost.

These forms of selection may be inputted preliminarily in the data base, so as to constitute a part of the function thereof.

While the criterion for selecting antimicrobial agent may be settled by a simple choice from the experiences accumulated over a long term, it is favorable that the criterion may permit to be revised under reference to the rate of success after accumulation of an amount of data in the early stage in which not so much data have yet been accumulated in the data base.

By performing the selection of antimicrobial agent in the manner as above, an antimicrobial agent which is at the most adapted to the microbial biota of the objective system can be selected in an assured way within a brief time.

By the process for effecting antimicrobial treatment, the objective system is treated with the antimicrobial agent selected as above, wherein the selection of the antimicrobial agent may be repeated either at a regular interval or at any voluntary occasion.

For instance, the process may be proceeded in such a manner that a sample containing microbes is collected periodically or at a voluntary occasion from the objective system and the microbial biota of the collected sample is analyzed based on base sequence of DNA in the manner as described above, whereupon the result of this analysis is compared with the result of the preceding analysis to watch any variation in the effect of the antimicrobial agent by the variation in the microbial biota. When no significant change is recognized in the microbial biota and an effect of the antimicrobial agent is noted, the antimicrobial agent presently on use is succeedingly employed. On the other hand, when a tendency to appearance of a microbe resistant against the antimicrobial agent presently on use is recognized, search in the data base for search for microbe is performed in order to pick up and select an antimicrobial agent effective for said microbe found to have said tendency to appearance. For instance, when an increase in the population of certain specific microbe (or DNA) is confirmed together with a marked increase in the formation of cling substance in an objective system in which certain antimicrobial agent(s) were used succeedingly, it is to be judged that a tendency to appearance of a microbe exhibiting resistance against the antimicrobial agent used is recognized, if the said microbe (or said DNA) has been registered in the data base as having significant resistance against the said antimicrobial agent or if the said antimicrobial agent is found to have a resistance against the said antimicrobial agent from experiment, assuring presence of certain causal relation between the variation in the microbial biota and the increase in the resistance of the microbe against the antimicrobial agent. In this manner, it is possible to perform the antimicrobial treatment using an antimicrobial agent which is regarded as being optimum at that point of time, by repeating the analysis of the microbial biota and the selection of antimicrobial agent.

By the process for effecting antimicrobial treatment, it is able to judge, upon occurrence of trouble in an objective system caused by growth of microbe, whether the cause therefor is based on appearance of a reagent-resistant microbe or not, within a brief time. Therefore a countermeasure for the trouble can promptly be taken by changing the antimicrobial agent to a new pertinent one for the cause of appearance of a resistant microbe or by revising the actual way of application of the antimicrobial agent (application concentration, manner of application etc.) or correcting the environmental condition of the objective system for other causes.

The process for effecting antimicrobial treatment can be carried out by incorporating, in combination, experimental results of, for example, sterilizing experiment, growth inhibiting experiment and microbe cell counting, which have not been recorded in the data base.

○ Method for monitoring antimicrobial treatment effect (reference aspect)

The method for monitoring antimicrobial treatment effect permits to monitor the effect of antimicrobial agent based on base sequence of DNA, by watching perodically or at a voluntary occasion any variation in the microbial biota in the objective system to which the antimicrobial agent has been applied.

Namely, a sample containing microbes is collected from the objective system periodically or at a voluntary period after application of the antimicrobial agent to the objective system and the microbial biota of the sample is analyzed based on base sequence of DNA as mentioned above, whereupon the result of this analysis is compared with the result of the preceding analysis to watch any variation in the effect of the antimicrobial agent by the variation in the microbial biota. When no significant variation in the microbial biota is recognized and the effect of the antimicrobial agent is admissible, the antimicrobial agent presently on use is used succeedingly. When a microbe not detected in the preceding analysis of the microbial biota is newly detected, collation is made with the data base as to whether or not the new microbe is non-susceptible or resistant to the antimicrobial agent presently on use. If the new microbe is susceptible to the antimicrobial agent, watching is continued as before, assuming that the antimicrobial agent is still effective for this microbe. If the new microbe is not susceptible or resistant to the antimicrobial agent, collation is made with the data base to search for antimicrobial agents effective for this new microbe in the manner described above to select an effective antimicrobial agent, whereby any trouble due to microbe can easily and assuredly be prevented.

By monitoring the antimicrobial effect in the manner as above, a precautional countermeasure can be taken preliminarily before, for example, a reduction in the antimicrobial effect or any trouble is brought about in the objective system. Thus, in the method for monitoring antimicrobial treatment effect, the monitoring means and the criterion for judging alteration of antimicrobial agent are provided by the analysis of the microbial biota and by the data base for search for antimicrobial agent, respectively.

○ Method for inhibiting slime formation in paper manufacturing process courses (reference aspect)

The method for inhibiting slime formation in paper manufacturing (in the following, sometimes referred to simply as the "slime formation inhibiting method") is directed to its application in paper manufacturing process courses, wherein no limitation is given to the kind of paper, specific process of paper manufaturing and so on. In paper manufacturing factory, a practice of slime control treatment has widely been employed, using a white water treating agent containing, as the main component, 2,2-dibromo-3-nitrilopropionamide (in the following, sometimes abbreviated to DBNPA). The slime formation inhibiting method can be applied adaptively to the process courses of such a paper manufacturing factory.

There is no special restriction as to the slime control agent to be used in the slime formation inhibiting method permitting thus use of known slime control agents, and any slime control agent adapted for each specific process course can be selected and used as a white water treating agent or as a microbe origin treating agent. The white water treating agent and the microbe origin treating agent may be composed of constituents which may be made of organic compound or of inorganic compound. These constituents of the slime control agent may be made of a single reagent or be made of a composite reagent in which a plurality of agents are combined together. Also, there is no restriction in the form of slime control agent used and it may be in a form of, for example, liquid agent, flowable agent or hydrated agent. There is also no restriction for combimation of white water treating agent with microbe origin treating agent and, thus, even a combination not bringing about a synergistic effect may be permitted.

As concrete examples of the slime control agent, there may be recited those based on inorganic and organic compounds as given below:

Slime control agents based on inorganic compound:

clorine, hypochlorites, chlorine dioxide, chlorinated cyanuric acid, chlorinated hydantoin, bromine, reaction product of hypochlorite with bromide ion and so on.

Slime control agents based on organic compound:

1) Surface active agents based on quaternary ammonium salt, such as didecyldimethylammonium chloride (in the following, sometimes abbreviated to DDAC) and so on.
2) Isothiazolones, such as 5-chloro-2-methyl-4-isothiazolin-3-one, 2-methyl-4-isothiazolin-3-one, 5-chloro-2-n-octylisothiazolin-3-one and 1,2-benzo-isothiazolin-3-one, and metal complex salts of them.
3) Thiocyanates, such as methylene bisthiocyanate and so on.
4) Bromoacetates, such as 1,4-bis(bromoacetoxy)-2-butene, 1,2-bis(bromoacetoxy)ethane and bis(bromo-acetoxy)propane.
5) Bromocyano compounds, such as 2,2-dibromo-3-nitrilopropionamide and 1,2-dibromo-2,4-dicyano-butane.
6) Bromonitro compounds, such as 2,2-dibromo-2-nitro-1-ethanol, 2-bromo-2-nitropropane-1,3-diol, β-bromonitrostyrene and 2,2-dibromo-3-nitrilopropionamide (DENPA).
7) Oximes, such as monochloroglyoxime, dichlorogly-oxime, 2-(p-hydroxyphenyl)glyoxyhydroximoyl chloride, α-chlorobenzaldoxime and α-chlorobenzaldoxime acetate.
8) Aldehydes, such as o-phthalaldehyde, glutaraldehyde and formaldehyde.
9) Others, such as 2,2-dichloro-1,2-dithiol-3-one, 2,4,5,6-tetrachloroisophthalonitril, 3,3,4,4-tetrachlorohydroxythiophene-1,1-dioxide

In a paper manufacturing process course including a plurality of origins of occurrence of a microbe having resistance against antimicrobial agents used, it is favorable to select a microbe origin treating agent adapted for each of the origins and to apply the selected treating agent to the corresponding origin. In the case where a plurality of resistant microbes resistant against the white water treating agent are present, it is favorable to select a microbe origin treating agent having antimicrobial activity for each of the resistant microbes and to apply the selected origin treating agent to the origin. Even in the case where a slime control agent has been used for the purpose of preservative treatment or prevention of denature, as in the systems for preparing papermaking reagents, such as the starch preparation system and dyestuff system, it is favorable to select a microbe origin treating agent to apply it additionally to the origin, so long as such system includes a site of origin of microbe occurrence.

In the method for inhibiting slime formation the microbial biota of the collected sample is analyzed based on base sequence of DNA, whereupon a slime control agent which is at the most adapted to the microbe detected by the analysis is selected by search in the data base. For the analysis of the microbial biota and for the selection of slime control agent, the procedures described in the paragraph of method for selecting antimicrobial agent for the microbe analyzing step and for the antimicrobial agent selecting step can be applied as such. In the following description of the slime formation inhibiting method, the "data base for search for treating agent" corresponds to the data base for search for antimicrobial agent used in the method for selecting antimicrobial agent.

The slime formation inhibiting method realizes first, as the microbe analyzing step, to collect samples from a paper manufacturing process course where slime formation inhibiting treatment is carried out using a slime control agent (white water treating agent). Then, the microbial biota of each sample is analyzed by a biota analysing method based on base sequence of DNA. Here, every microbe constituting said microbial biota is searched by referring to the microbe name or the accession number (corresponding to the microbe name) by the method given above. Usually, the series of the above examination operations may be completed within a period of 2 - 7 days. In the microbe analyzing step, only the identification of microbe is performed and the quantitative analysis of its population may be omitted.

While there is no restriction as to the site for collecting the sample and samples can be collected at every voluntary location in paper manufacturing process courses, samples are collected at two or more locations, preferably at 5 - 20 locations. The greater the number of locations of sample collection, the more accurately the origin of occurrence of resistant microbe can be determined.

Concretely, samples of slime, white water and internal liquor in tanks and vessels may be collected from process courses, for example, pulp raw material preparation system composed of CGP (chemical ground pulp) pulper, CGP chest, LBKP (laubholz bleached kraft pulp) pulper, LBKP chest and so on; broke system composed of wet broke pulper, wet broke chest, dry broke pulper, dry broke chest and so on; paper-masking chemical preparation system composed of sizing agent tank, aluminum sulfate tank, dyestuff tank, slime contol agent tank, internal starch liquor tank and so on; stock preparation system composed of mixing chest, machine chest, stuff box and so on; white water circualtion system composed of paper machine, screen unit, machine inlet, saveall, white water silo and so on; and white water recovering system composed of dehydrator, clear white water pit and so on.

Then, judgement is made, in a resistance discriminating step, as to whether or not the microbe detected in the microbe analysing step mentioned above is resistant against the slime control agent (white water treating agent) now on use. For example, search in the data base is performed using, as the search key, the microbe detected in the microbe analysing step or the slime control agent now on use, whereupon it is judged that the microbe under search is not resistant aganst the slime control agent now on use, when effective concentration of the slime control agent (white water treating agent) now on use for the microbe under serach is recorded in the data base, or is judged to be resistant, when no record is found as to the effective concentration. It is also possible to judge presence or absence of the resistance from, for example, known literatures or from the results of culture experiments. Further, it is possible to judge for a microbe to have a resistance against a white water treating agent, when a tendency to growth of this microbe is recognized even though the microbe is held under treatment with the white water treating agent, by comparing the result of analysis with that of the preceding analysis.

Thereafter, judgement is made as to the site of origin of occurrence of the resistant microbe that the location of collection of the sample that showed highermost proportion of existence of the resistant microbe is the site of origin of occurrence of such microbe. The site of origin of occurrence of a microbe may occasionally be present in plural numbers. In the case where analysis of the microbial biota of the sample is incorporated in the microbe analyzing step, the constitutional proportion or the content of each microbe has already been determined, so that a renewed determination of the proportion of existence of the resistant microbe is unnecessary and the site of origin can be judged from the already determined proportion of existence. On the other hand, in the case where only the kind of the microbe is analyzed in the above microbe analyzing step, the site of origin of occurrence of the resistant microbe is judged from the result of determination of the proportion of existence performed by the method as given above for the sample in which the resistant microbe is detected.

Subsequently, search in the data base for search for treating agent is carried out in a slime control agent selecting step using, as the search key, the microbe that is judged in the above resistance judging step as being resistant against the slime control agent, in order to pick up the slime control agents of which effective concentrations for the resistant microbe are recorded in the data base. Here, the slime control agents to be searched can be limited to a more narrow extent when environmental condition etc. are used as the search key. The result of picking up may be outputted on an output device, such as a display. For the case where only one slime control agent was picked up, this slime control agent constitutes the selected one and, for the case of a plurality of slime control agents, selection is effected among them. The manner for selecting a slime control agent to be used actually as the microbe origin treating agent among the picked up ones is arbitrary and can be performed, for example, by human visual choice, by automatic choice by computer and so on. Concrete manner of selection may include, for example, the following cases:

1) A slime control agent exhibiting a minimum effective concentration which is at the most low for the resistant microbe is selected.
2) A slime control agent, by which the value

$$\text{(minimum effective concentration)} \times \text{(price)}$$

is the lowest for the resistant microbe, is selected. Here, selection can be effected taking into account of the treating cost.

These forms of selection may be inputted in the data base for search for treating agent, so as to constitute a part of the function thereof.

While the criterion for selecting the slime control agent may be settled by a simple choice from the experiences accumulated over a long term, it is favorable that the criterion may be permitted to be revised especially in the early stage in which not much data have yet been accumulated in the data base, taking into account of the rate of success upon each accumulation of data.

Then, in an origin treating agent adding step, the new slime control agent selected in the above slime control agent selecting step, namely, an origin treating agent which is different from the white water treating agent presently on use, is added to the site of origin of occurrence of the resistant microbe judged as resistant in the above microbe origin determining step.

By the slime formation inhibiting method , the microbe origin determining step may be omitted. Here, the new slime control agent (the origin treating agent) selected in the slime control agent selecting step is added to the location of collection of the sample that showed high proportion of existence of the resistant microbe judged in the resistance discriminating step.

By the technique as described above, it is able to select a slime control agent most adapted to the resistant microbe within a brief time in an assured way, together with attainment of minimization of the amount of the slime control agent to be used, since such slime control agent can be added to the site of origin of occurrence of the microbe.

The slime formation inhibiting method permits to repeat certain series of steps including the microbe analysing step and so on at a regular interval or at any volumtary occasion.

For instance, samples are collected at a regular internal or at a voluntary occasion from paper manufacturing process courses and the microbial biota of each sample is analyzed based on base sequence of DNA, whereupon the result of this analysis is compared with that of the preceding analysis to watch any variation in the effect of the slime control agent used by the variation in the microbial biota. When no significant change in the microbial biota is recognized and an effect of the slime control agent is to be admitted, this slime control agent is used succeedingly. On the other hand, when a decrease in the proportion of existence of a resistant microbe is found, addition of the origin treating agent may be stopped. When a new resistant microbe is further detected, a new origin treating agent for this new microbe is selected to use.

In this manner, slime formation inhibiting treatment can be performed using a slime control agent which is at the most adapted at that point of time by carrying out the analysis of the microbial biota and the selection of the origin treating agent repeatedly.

By the slime formation inhibiting method , it is able to judge within a brief time whether or not a trouble occured due to growth of microbe in a paper manufacturing process course held under treatment with a slime control agent is caused from occurrence of a microbe resistant against the slime control agent presently on use, so that a prompt responce to the trouble can be made by changing the slime control agent for the cause due to occurrence of resistant microbe or, otherwise, by taking pertinent steps as to the practical manner of application of the slime control agent (such as the application concentration and practical manner of application) and as to the alteration of environmental condition appeared, since these may be assumed to be the other cause.

It is able in the slime formation inhibiting method to make use of a above-data base for search for treating agent as given above for selecting the white water treating agent. For example, a slime control agent to be used as the white water treating agent can be selected, by performing search in the data base using, as the search key, the microbe found in the paper manufacturing process course as the dominant microbe, to pick up slime control agents and selecting among the picked up ones one or more slime control agents to be used as the white water treating agent.

The slime formation inhibiting method permits to make use of, in combination, the results of, for example, preservative experiments, growth inhibiting experiments and microbe cell counting, which have not yet been recorded in the data base, for the selection of the slime control agent.

○ Method for analyzing cling substance on products of manufacture in paper manufacturing process (reference aspect)

The method for analyzing cling substance on products of manufacture in paper manufacturing process (in the following, referred to sometimes simply as the analyzing method) can be applied to paper products produced in paper manufacturing process, wherein there is no limitation in the kind of paper, the manufacturing technique and so on. The paper manufacturing process is in general composed of process courses, for example, the stock preparation course in which the raw material for papermaking is prepared by adding fillers and reagents to pulp, the papermaking course in which paper is produced on a paper machine and the coating course in which the surface of paper is coated with coating colour or other material(s) to improve adaptability to printing. The analyzing method can be applied to paper products produced through these process courses.

There is no special restriction in performing the method for extracting DNA originated from microbe(s) which cause the cling substance on paper products and the method can be performed, for example, in such a way that the cling substance (defect) or the portion of the paper product including the cling substance is cut out and is scissored, whereupon the scissored product is immersed sufficiently in an extracting liquor, such as a buffer solution, followed by processing by a physical treatment, such as mechanical crushing, or a chemical treatment, such as treatment with a surfactant or with an enzyme, solely or in combination, in order to extract the DNA originated from the cells of the microbes in the cling substance. Here, the extraction of DNA can be realized even if the microbes are extinct.

As concrete techniques for the above physical treatment, there may be exemplified ultrasonic crushing, freezing/thawing and homogenization using micronous glass beads or mineral beads. As concrete techniques of the above chemical treatment, there may be exemplified enzymatic treatments, for example, those using bacteriolysing enzymes, such as lysozymes; polysaccharide decomposing enzymes, such as cellulase and alginate lyase; and protein decomposing enzymes, such as protease and peptidase; and those using surfactants, for example, anionic surfactants, such as sodium lauryl sulfate and so on; and nonionic surfactants, such as Triton-X 100 and so on. By the physical or chemical treatment, DNA are obtained in a dissolved form. They may preferably be further purified. For purifying the DNA, known techniques can be employed, for example, desolvatic precipitation by organic solvents, such as ethanol and isopropanol; and adsorption on glass beads or resin particles.

For detecting the characteristics of each microbe and the constitutional proportion thereof based on the base sequence of the extracted DNA, the relative content of the base sequence that permits identification of the microbe is determined. For identifying the dominant microbe, it is assumed that the microbe of which relative content determined as above was high constitutes the dominant microbe. Such analysis of the microbial biota can be performed by making use of the practical procedures of the microbe analyzing step described for the above method for selecting antimicrobial agent as such. Namely, there may be used as the DNA permitting identification of each microbe, any DNA segment so long as all the microbes to be analyzed have this DNA segment, wherein preference is given to such DNA segment for which a data base capable of identifying the microbe from the base sequence has already been constructed, such as those which code ribosomal RNA (rDNA), for example, 16S rDNA, 18S rDNA and 23S rDNA, and spacer sequences for them, and gyrE or the like.

While it is possible to determine the kind of DNA and the relative content thereof by dot hybridization or by an efficient hybridization method using DNA chip, using the extracted DNA directly as the target, it is usual to employ an in vitro amplification by, for example, polymerase chain reaction (PCR), to amplify the DNA to attain the determination. For example, for 16S rDNA, this can be amplified in a mixture using all the extracted DNA as the template by means of universal primers designed for a base sequence present commonly in most of microbes.

Since 16S rDNA is extracted as a mixture of two or more originated from two or more microbes that caused the cling substance, the mixture of 16S rDNA is subjected to an amplification to determine the constitutional proportion of each DNA by the amplified mixture, wherein it is made possible to identify each microbe name based on the detected corresponding DNA, whereby the dominant microbe and microbial biota of the cling substance can be identified. For determining the constitutional proportion of each DNA, known techniques may be utilized, such as the techniques based on electrophoresis, for example, DGGE (denatured gradient gel electrophoresis; Muyzer et al, Applied and Environmental Microbiology 59, 695 - 700 (1993)), TGGE (temperature gradient gel electrophoresis; Eichner et al, Applied and Environmental Microbiology 65, 102 - 109 (1999)) and SSCP (single strand comformational polymorphism; Schwieger et al, Applied and Environmental Microbiology 64, 4870 - 4876 (1998)) and isolation technique, such as TRFLP (terminal restriction fragment length polymorphism; Liu et al, Applied and Environmental Microbiology 63, 4516 - 4522 (1997)). A rough calculation of the constitutional proportion of microbes can be attained by determining the amount of amplified product intrinsic to each microbe and calculating the proportion thereof relative to the entire amplified product. By using the above techniques based on electrophoresis or TRFLP, the amplified products can be detected, for example, as the intensity of the band of the DNA or as the fluorescence intensity corresponding to the TRF, and the constitutional proportion of the microbes can be detected by the ratio of such intensities.

The microbial biota can also be identified by subjecting the amplification product to a random cloning using an established host vector system, such as Escherichia coli (Dunbar et al, Applied and Environmental Microbiology 65, 1662 - 1669 (1999)), and analyzing the genes of the resulting clones and their constitutional proportions.

Identification of a microbe by DNA of its gene can be achieved by determining the base sequence of the DNA by, for example, DNA sequencer, and comparing the analyzed base sequence with the corresponding data in the data base, whereby taxonomical localization and microbiological nomenclature of the microbe having corresponding DNA can be attained. For an analysis sample in which DNA of one single kind of microbe occupies nearly about 90 % of the entirety, the base sequence of the dominant microbe can be determined by direct determination of the base sequence of the amplified produt. Different DNA molecules may be isolated by, for example, electrophoresis, wherein the corresponding band of DNA molecule is collected from the gel and is subjected to amplification again by PCR, whereupon the base sequence of each DNA can be determined using DNA sequencer.

For the data base for search for microbes, there may be enumerated, for example, public DNA data bases, such as GenBank, EMBL and DDBJ, as well as Ribosomal Database Project installed in the University of Michigan. Operation of search can be realized in a brief time efficiently by existing program, such as FASTA and BLAST and the like. It is also possible to search in a commercial data base, such as MicroSeq 16S rDNA Sequence Database (trademark, of Applied Biosystems Japan, LTD.), using a commercially available software, such as MicroSeq Analysis Software (trademark, of Applied Biosystems Japan, Ltd.). It is also permitted to construct such a data base privately for utilization. When microbe name of a microbe is not able to identify from the data base, it may be assumed that the microbe is a near related one having nearmost homology with that in the data base or that the microbe belongs to a new species.

By analyzing the microbial biota or the dominant microbe in the cling substance in the manner as above, the names of the microbes, the constitutional proportions thereof, the dominant microbe and so on can easily be identified within a brief time in an assured manner, without having resort to culture of microbe.

○ Method for inspecting the causal basis of formation of cling substances

The method for inspecting the causal basis of formation of the cling substances on paper products according to the present invention consists in a technique in which the microbial biota or the dominant microbe in the cling substance identified by the method for analyzing cling substance as detailed above is compared with the microbial biota or the dominant microbe in the slime appearing in a paper manufacturing process course, wherein the slime of which microbial biota or the dominant microbe that is in accord with or close to that in the cling substance is judged to be the causal slime for the formation of cling substance. The microbial biota or the dominant microbe can be identified in the same manner as in the identification of the microbial biota or the dominant microbe in the cling substance using the base sequence of DNA as the analysis parameter, as given above.

There is no restriction in the location for collecting the sample of slime and it is permitted to collect samples at any location in paper manufacturing process courses, wherein samples may favorably be collected at two or more locations, preferably at 5 - 20 locations. The greater the number of locations of sample collection, the more accurately the causal slime that caused the cling substance can be determined.

Concretely, samples of slime may be collected from process courses, for example, pulp raw material preparation system composed of CGP (chemical ground pulp) pulper, CGP chest, LBKP (laubholz bleached kraft pulp) pulper, LBKP chest and so on; broke system composed of wet broke pulper, wet broke chest, dry broke pulper, dry broke chest and so on; papermaking chemical preparation system composed of sizing agent tank, aluminum sulfate tank, dyestuff tank, slime contol agent tank, internal starch paste tank and so on; stock preparation system composed of mixing chest, machine chest, stuff box and so on; white water circualtion system composed of paper machine, screen unit, machine inlet, head box, saveall, white water silo and so on; and white water recovering system composed of dehydrator, clear white water pit, tubes in CP (consistency profiling control system) and so on; and elements and instruments around the paper machine, such as wire, wheel and so on. It is possible to roughly presume the possible site of origin of occurrence of slime based on the analysis of microbial biota or the dominant microbe, by collecting samples of, for example, white water, tank inner liquor etc., and performing the analysis by the above-mentioned analyzing method, whereupon collection of sample is focussed mainly to such presumed possible locations.

The method for inspecting the causal basis of slime formation according to the present invention can attain detection of the location where the causal microbe for the cling substance crowds at high population or the site of origin of occurrence thereof in an easy and assured manner, since the slime that caused the formation of cling substance is identified by comparing the result of analysis of the microbial biota or the dominant microbe in the cling substance with that in the slime occurred in paper manufacturing process courses.

○ Method for controlling microbes

The method for controlling microbes occurring in paper manufacturing process courses according to the present invention consists in a technique in which the location of occurrence of the slime that caused the cling susbstance is detected in the manner as given in the above method for inspecting the causal basis of formation of cling substance, whereupon an antimicrobial/preservative treatment is performed at the so-detected location of occurrence of the slime.

The antimicrobial/preservative treatment can be performed by, for example, applying to the location of occurrence of the slime a slime control agent having antimicrobial/preservative activity to the microbes, especially the dominant microbe, constituting the slime. The slime control agent to be applied can be decided by, for example, performing antimicrobial experiment. It is also possible for the case where the name of the causal microbe has been identified to use a chemical which is known to have an antimicrobial/preservative activity to the microbe. It is also possible to select the slime control agent from the data base for search for antimicrobial agent by the method given in the antimicrobial agent selecting step described in the paragraph of the method for selecting antimicrobial agent.

By the method for controlling microbes as described above, occurrence of defect on paper product can be prevented efficiently and rationally at a lower cost, without relying on empirical or circumstantial judgement, since the method provides for a technique, in which a treating agent effective for the causal microbe is selected in an assured manner and which can afford to realize antimicrobial/preservative treatment focussed substantially onto the site where the causal microbe densely populates or vigorously propagates, using lowest necessary amount of the treating agent.

As described above, it is able by the method for selecting antimicrobial agent to attain selection of the optimum antimicrobial agent simply within a brief time in an assured manner, since the method provides for a technique in which the microbial biota of the sample is analyzed based on base sequence of DNA and an industrial antimicrobial agent adapted at the most to the microbial biota is selected by making use of a data base.

The process for effecting antimicrobial treatment can afford to effect antimicrobial treatment efficiently by selecting an optimum industrial antimicronial agent in accordance with each specific microbial biota of the objective system within a brief time, since the process provides for analyzing the microbial biota of the objective system based on base sequence of DNA and using an industrial antimicrobial agent adapted at the most to the microbial biota selected from a data base.

The method for monitoring antimicrobial treatment effect can afford to judge in a simple but assuered manner within a brief time whether or not the activity of the antimicrobial agent has been revealed to thereby enable to prevent occurrence of troubles caused by growth of microbes beforehand, since the method provides for analyzing the microbial biota of the objective system based on base sequence of DNA and watching the effect of the antiicrobial agent based on the variation in the microbial biota detected by the analysis.

The slime formation inhibiting method can afford to select a slime control agent which is adapted at the most to the resistant microbe within a brief time in an assured manner and to attain an efficient inhibition of slime formation with minimum amount of the slime control agent, since the method provides for analyzing the microbial biota of the objective system based on base sequence of DNA and selecting, when occurrence of a microbe which is resistant against the antimicrobial agent is recognized, a slime control agent which is at the most adapted to the resistant microbe from a data base to apply it to the site of origin of occurrence of the resistant microbe.

The method for analyzing cling substance on a paper product in paper manufacturing process can afford to identify the causal microbe that caused the defect on the paper product, in a simple and assured manner, since the method provides for extracting the DNA originated from the microbe in the cling substance and analyzing the microbial biota or the dominant microbe of the cling substance by using the base sequence of the extracted DNA as analysis parameter.

The method for inspecting the causal basis of formation of cling substances on paper products in paper manufacturing process courses according to the present invention can afford to identify the site of origin of occurrence of the causal microbe that caused the defect on the paper product, in a simple and assured manner, since the method provides for extracting the DNA originated from the microbe in the cling substance, analyzing the microbial biota or the dominant microbe of the cling substance by using the base sequence of the extracted DNA as analysis parameter and comparing the so analyzed microbial biota or the dominant microbe with that of the slime occurred in paper manufacturing process courses to determine the slime that caused the cling substance.

The method for controlling microbes occurring in paper manufacturing process courses according to the present invention can afford to determine the location of occurrence of the microbe that caused the defect on the paper product in a simple and assured manner and to suppress occurrence of defect on paper products in a simple and assured manner, since the method provides for extracting the DNA originated from the microbe in the cling substacne, analyzing the microbial biota or the dominant microbe of the cling substance by using the base sequence of the extracted DNA as analysis parameter, comparing the so analyzed microbial biota or the dominant microbe with that of the slime occurred in paper manufacturing process courses to determine the location of occurrence of the slime that caused the cling substance and performing an antimicrobial/preservative treatment at the so-determined location of occurrence of the slime.

Below, the description is directed to the process for effecting antimicrobial treatment according to the present invention with reference to the drawings appended.

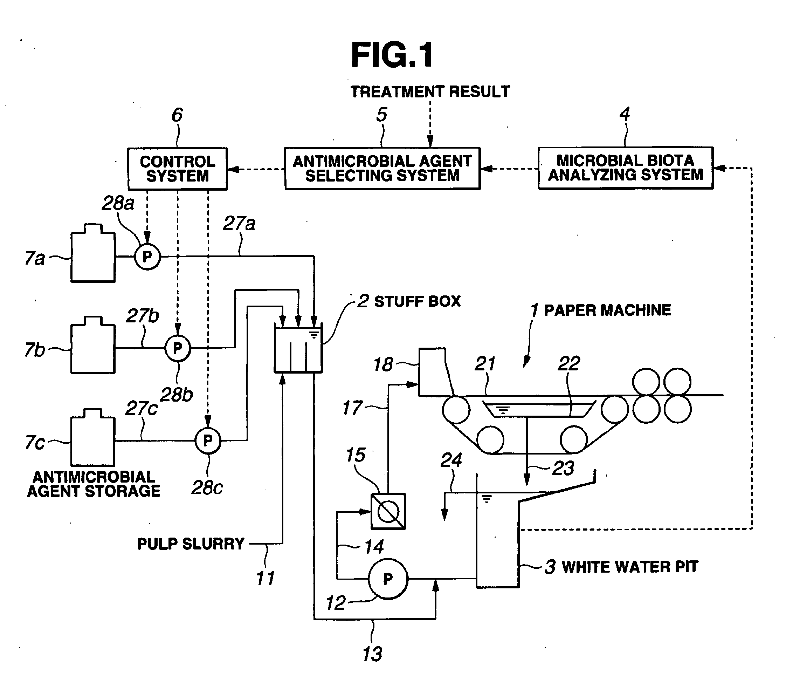

Fig. 1 is a flow diagram of the apparatus for paper manufacturing, to which the process for effecting antimicrobial treatment is to be applied, shown in an embodiment of antimicrobial treatment of white water system.

In the apparatus shown in Fig. 1, the numerals indicate, 1: paper machine, 2: stuff box, 3: white water pit, 4: microbe biota analysing system, 5: antimicrobial agent selecting system, 6: control system, 7a, 7b: antimicrobial agent storage disposed in plural numbers.

On the apparatus shown in Fig. 1, paper is produced in the manner as follows: A pulp slurry is supplied via a line 11 into the apparatus under adjustment of flow rate at the stuff box 2 and is guided by a pump 12 via lines 13 and 14 to a screen unit 15, where contaminant materials are removed, before being sent via a line 17 to the inlet 18. The pulp slurry is then supplied from the inlet 18 to the wire part 21 of the paper machine 1. The white water passed through the wire part 21 is received in a saveall 22 and is collected via a line 23 in the white water pit 3. A part of the white water in the white water pit 3 is admixed to the pulp slurry through the line 14 for reuse under recirculation. The remainder is discharged out via a line 24. The web of damp pulp formed on the wire part 21 is processed through subsequent process courses, such as drying (not shown) etc., into a paper product.

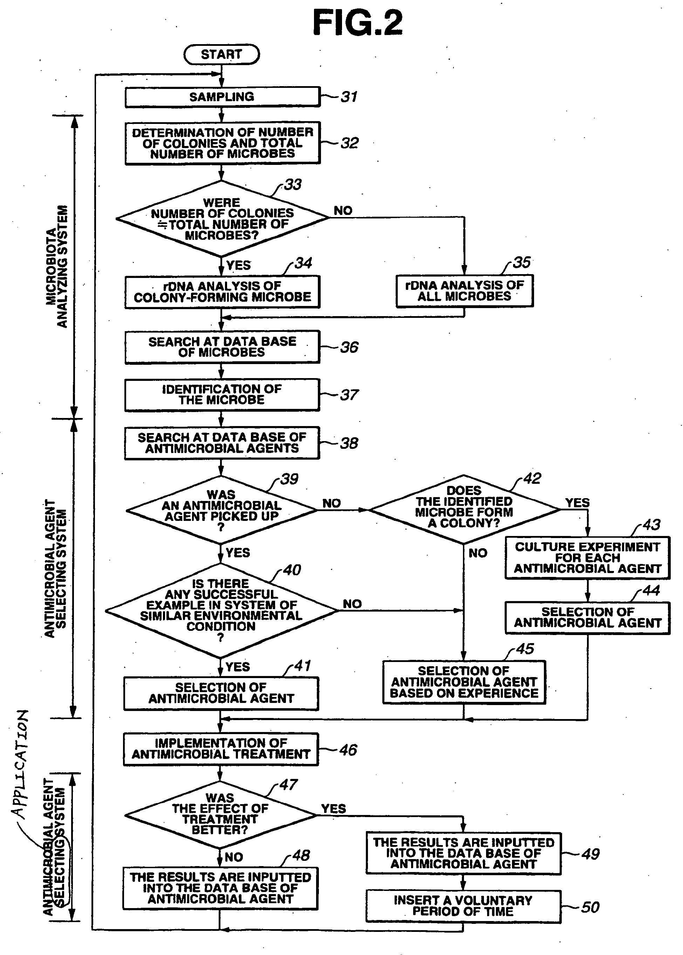

For performing the antimicrobial treatment in the apparatus as given above, a sample of white water containing microbes is collected from the white water pit 3, for which analysis of microbial biota is carried out in the microbial biota analyzing system 4. Based on the result of this analysis, an anitimicrobial agent is selected in the antimicrobial agent selecting system 5. The analysis of the microbial biota in the microbial biota analysing system 4 and the selection of the antimicrobial agent in the antimicrobial agent selecting system 5 are performed according to an operation flow schedule, for example, that shown in Fig. 2.

The result of operation of the antimicrobial agent selecting system 5 is inputted into the control system 6, whereby the selected antimicrobial agents are added to the stuff box 2 from the antimicrobial agent storages 7a, 7b, .... via each reagent injection line 27a, 27b, ...., so that each designated antimicrobial agent will reach a designated concentration.

By maintaining the admixed antimicrobial agent dissolved in the white water and in the pulp slurry under recirculation in the paper manufacturing apparatus, the entire apparatus will be held under antimicrobial treatment. The adjustment of the concentration of antimicrobial agent may be realized automatically by controlling the amount of addition thereof by inputting corresponding signals to each actuator of the pump 28a, 28b .... from the control system 6.

The sampling of white water from the white water pit 3 may be carried out at regular interval or at an arbitrary occasion, whereupon the analysis of the microbial biota of the sample and the selection of the antimicrobial agent are performed. By this, an antimicrobial agent which is at the most adaptive at this point of time can be selected and the antimicrobial treatment can be realized at the highest effectively.

While Fig. 1 shows the case where the sample is collected from the white water pit 3, sampling may be effected at any other location, such as the screen unit 15, the machine inlet 18, the saveall 22 or so on. While Fig. 1 shows three antimicrobial agent storages 7a, 7b ..., two or four or more may be permitted.

Fig. 2 is a flow diagram showing the operation flow schedule in the microbial biota analysing system 4 and in the antimicrobilal agent selecting system 5 for an embodiment in which a data base, that stores data of antimicrobial agents at concentrations effective for microbes under each specific environmental condition so as to permit selection of antimicrobial agents within a more narrow extent, is employed.

According to the flow diagram of Fig. 2, a sample containing microbes is collected from the objective system in the step 31 and, in the step 32, the counts of colonies brought from the sample and the total number of microbe cells are determined. The total number of microbe cells may be determined by a known technique, for example, by counting the cells after stained with, for example, acridine orange or DAPI, directly under a fluorescence microscope. In the case where the results may be presupposed from the environmental condition for an objective system for which experiments have been carried out many times, the procedures of step 32 can be omitted.

In the step 33, comparison of the number of colonies with the total number of microbe cells is performed. If both the numbers are nearly equal, analysis of rDNA of the colony-forming microbe is carried out in the step 34. On the contrary, if both numbers are unequal, rDNA analysis for all the microbes including non-colony-forming microbes is performed in the step 35.

Then, in the step 36, search in the data base for serach for microbes is made based on the base sequence determined in the above analysis. Here, search may be performed in a plurality of data bases for search for microbe. Based on the result of the search, each of the microbes is identified in the step 37 (determination of the microbial biota). If, in this case, identification of microbe name is not permitted, a private microbe number may be assigned to the microbe.

In the subsequent step 38, search in the data base for search for antimicrobial agent is performed using the microbe name that is identified in the step 37, the accession numer or the privately assigned microbe number as the search key. If antimicrobial agents were picked up in the step 39, selection of the antimicrobial agent may be limited into a more narrow extent by using the environmental conditions as the search key in the step 40 and, when sucessful examples are found with the searched antimicrobial agent, this antimicrobial agent is selected as the treating agent to be used for the antimicrobial treatment in the step 41. Here, the concentration of the antimicrobial agent for the treatment is also determined. When a plurality of antimicrobial agents are put up as candidates, the selection may be done taking into account of the cost performance and so on.

In case no antimicrobial agent is picked up by search in the data base for search for antimicrobial agent in the step 39, the operation schedule is carried over to the step 42 where the discrimination as to whether the microbe in question is a colony-forming microbe or not is made by reference to the result of the step 33, wherein, for the case where the microbe is a colony-forming microbe, a laboratory culture experiment thereof is carried out in the step 43 for examining the resistance to various antimicrobial agents, whereupon the selection is made based on the result of this culture experiment (step 44). When the microbe is judged to be a non-colony-forming microbe in the step 42, selection of antimicrobial agent is performed in the step 45 besed on, for example, the experiences in the past and so on.

Using the antimicrobial agent selected in the manner as above, the antimicrobial treatment is carried out in the step 46.

Then, the effect of the treatment is judged in the step 47 and, when the antimicrobial effect is not better, this result is inputted in the data base for search for antimicrobial agent (step 48), whereupon the operation schedule is returned to the step 31 of sample collection and the operations thereafter are repeated again. If the microbe identified thereby is the same as that identified previously, an antimicrobial agent different from that selected in the former operation is selected among those restricted in the step 40. When the result of analysis of DNA in the step 34 or 35 is identical with that in the former operation, the search in the data base for search for microbe in the step 36 may be omitted.

When the effect of the treatment is judged in the step 47 to be better, this result is inputted in the data base for search for antimicrobial agent (step 49), whereupon the operation schedules downstream from the step 31 are repeated, after a certain arbitrary interval has elapsed (step 50).

While, in the operation flow schedule shown in Fig. 2, selection of the antimicrobial agent is made by performing a culture experiment for colony-forming microbe in the case where no antimicrobial agent is permitted to be picked up in the step 39, it may be possible to effect the selection based on, for example, experiences in the past and so on.

By following the operation schedules given as above, the proportion of the case where the judgement of "no" is made in the steps 39 and 40 will successively be decreased as the data of the results are accumulated in the data base on the course of progress of experiences of the actual antimicrobial treatment while inputting the results in the data base, whereby an adaptive antimicrobial agent will eventually become able to be selected only by retrieving in the data base for search for antimicrobial agent.

Now, the description is directed to the method for inhibiting slime formation by reference to the appended drawing.

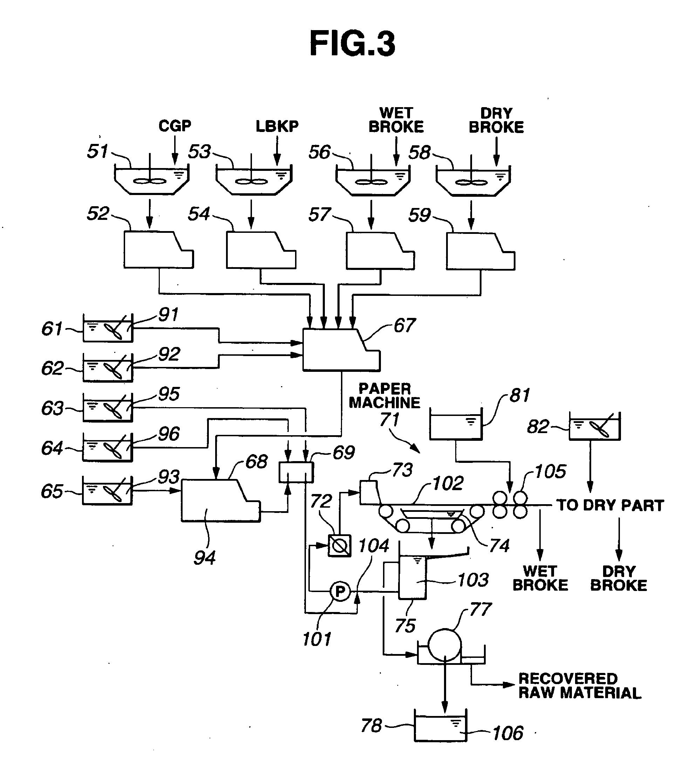

Fig. 3 is a flow diagram of paper manufacturing apparatus to which the method for inhibiting slime formation is to be applied.

In Fig. 3, the numeral 51 represents a CGP pulper, 52 is a CGP chest, 53 is an LBKP pulper and 54 is an LBKP chest, which constitute the pulp raw material preparation system.

The numeral 56 represents a wet broke pulper, 57 a wet broke chest, 58 a dry broke pulper and 59 a dry broke chest, which constitute the broke system.

The numeral 61 denotes a sizing agent storage, 62 an aluminum sulfate storage, 63 a dyestuff storage, 64 a white water treating agent storage and 65 an internal starch paste storage, which constitute the papermaking chemical-preparation system.

The numeral 67 denotes a mixing chest, 68 a machine chest and 69 a stuff box, which constitute the stock preparation system.

The numeral 71 denotes a paper machine, 72 a screen unit, 73 a machine inlet, 74 a saveall and 75 a white water silo, which constitute the white water circulation system.

The numeral 77 denotes a separator-thickener and 78 a clear white water pit, which constitute the white water recovery system. The numeral 81 indicates an industrial water pit and 82 represents an external starch paste storage.

On the apparatus shown in Fig. 3, paper is produced in the manner as follows:

The pulp as the raw material of paper is guided into the CGP pulper 51, LBKP pulper 53, wet broke pulper 56 and dry broke pulper 58 and is there disintegrated to form a slurry which is guided via the chests 52, 54, 57 and 59 into the mixing chest 67. Thereto are admixed a sizing agent 91 and aluminum sulfate 92 and, then, an inner starch paste 93 in the machine chest 68 to build up a pulp slurry 94. This pulp slurry 94 is guided into the stuff box 69, where a dyestuff 95 and a white water treating agent 96 are admixed thereto and the resulting slurry is sent under adjustment of the flow rate to the screen unit 72 by a pump 101, to remove there contaminant alien matter, before being introduced into the machine inlet 73.

The so-introduced pulp slurry 94 is guided from the machine inlet 73 onto wire part 102 of the paper machine 71. The white water separated from the wire part 102 is once received in the saveall 74 and is then collected in the white water silo 75. A part of the white water 103 accumulated in the white water silo 75 is mixed with the pulp slurry 94 in a line 104 and is reused under recirculation. The remainder of the white water is sent to the separator-thickener 77 where it is separated from solid matters and the thereby recovered raw materials are returnd back to the mixing chest 67 to reuse. The filtrate is collected in the clear white water pit 78 and is reused in the process courses. The pulp web formed on the wire part 102 is guided through the subsequent process courses in a press part 105, drying part (not shown) and so on, before being processed into the paper product. The brokes occurring in the press part 105 and in the drying part are transferred each as the wet broke and dry broke to the wet broke pulper 56 and to the dry broke pulper 58, respectively, to reuse.

In the paper manufacturing process for producing paper as given above, a white water treating agent 96, such as 2,2-dibromo-3-nitrilopropionamide (DBNPA), introduced from the white water treating agent storage 64 and retained dissolved in the pulp slurry 94 and in the white water 103 is circulated in the white water circulation system, whereby slime control in the white water circulation system is attained. Due to the re-utilization of the clear white water 106 collected in the clear white water pit 78, slime control is also effected in the process courses where the clear white water is reused.

From the paper manufacturing process courses held under treatment of slime control in the manner as above, microbe-containing samples are collected at arbitrary two or more locations and the microbial biota of each of the samples is analyzed by the method described previously based on base sequence of DNA (microbe analyzing step). The sampling may be effected at, for example, CGP chest 51, LBKP chest 53, wet broke chest 56, dry broke chest 58, sizing agent storage 61, dyestuff storage 63, internal starch paste storage 65, mixing chest 67, machine chest 68, white water silo 75, clear white water pit 78, industrial water pit 81 and external starch paste storage 82.

Then, in the resistance discriminating step, judgement is made as to whether or not the microbe detected in the above microbe analyzing step has a resistance to the white water treating agent 96 now on use. For example, search is performed in the data base for search for treating agent using the microbe detected in the above microbe analyzing step or the white water treating agent presently on use as the search key, whereby the microbe now on search can be judged as having no resistance to the antimicrobial agent now on use, when the data for the effective concentration of the antimicrobial agent now on use is found recorded for the antimicrobial agent now on use in the data base, but can be judged as having resistance to the antimicrobial agent now on use, when no such data is found recorded therein.

Subsequently, in the origin determining step, the sampling portion at which the collected sample showed higher proportion of exsistence of the resistant microbe judged in the resistance discriminating step as having resistance is judged to be the site of origin of occurrence of the microbe. For example, when the proportion of existence of a microbe resistant against DBNPA is the highest at the wet broke chest 56, it can be judged that the site of origin of occurrence of the microbe is the wet broke chest 56. There may be a case where a plurality of sites of origin of occurrence of the microbe are detected.

Thereafter, in the slime control agent selecting step, search is made in the data base for search for treating agent using the resistant microbe judged in the resistance discriminating step to have resistence as the seach key to pick up each slime control agent for which data of the effective concentration for the resistant microbe are found inputted. Here, it is preferable to input environmental condition or the like to restrict the extent of search for the slime control agent. When a plurality of slime control agents were detected as the candidate, decision is made to select a slime control agent to be used as the origin treating agent in such a manner that a slime control agent which exhibits lowermost effective concentration for the resistant microbe or a slime control agent for which the value

$$[(\text{minimum effective concentration}) \times (\text{price})]$$

is the lowest is selected.

Then, in the origin treating agent adding step, the new slime control agent selected in the slime control agent selecting step, namely, different from the white water treating agent now on use, is added to the site of origin of occurrence of the resistant microbe. For example, a slime control agent selected due to its effectiveness for the resistant microbe, such as HPGHC, namely, 2-(p-hydroxyphenyl)glyoxylohydroximoyl chloride, is added to the wet broke chest 56. The resistant microbe is thereby subjected to an antimicrobial treatment at its origin of occurrrence effectively, whereby slime formation is inhibited.

The sampling may be realized periodically or at an arbitrary interval repeatedly. When effect of the origin treating agent is recognized, application of the origin treating agent is stopped and ordinary treatment using the white water treating agent is regained. When no effect of the origin treating agent is recognized, the origin treating agent may be changed to another one or revision of the manner of application of the origin treating agent (for example, application concentration, application procedure etc.) or the environmental condition may be attempted. When a new resistant microbe is detected, corresponding new origin treating agent effective for the new microbe is selected to use.

By performing the analysis of microbial biota and the selection of origin treating agent repeatedly in the manner as above, a slime control agent which is at the highest adaptive at that point of time can be selected within a brief time in an assured manner with the advantage that the inhibition of slime formation can be performed using minimum amount of slime control agent due to permission of application to the site of origin of occurrence of the microbe.

THE BEST MODE FOR EMBODYING THE INVENTION

Below, the present invention is described by way of Examples and Reference Examples.

EXAMPLE 1

<< Experiment Example by a Laboratory Slime Formation Testing Apparatus >>

A torque type slime formation testing apparatus was used, which is described in Japanes Patent Kokai Hei 9-75065 A. The testing apparatus is constructed from a stationary outer cylinder and a rotatable inner cylinder disposed concentrically with the outer cylinder leaving a free space therebetween for allowing flow of water therein while rotating the inner cylinder so as to permit to grow slime formed on the surface of the inner cylider. To this testing apparatus, an artificial white water (containing 142 mg/liter of soluble starch and 11.6 mg/liter of ammonium sulfate and having a pH of 7.0) was supplied continuously at a temperature of 30 ˚C so as to maintain a residence time in the apparatus of 20 minutes, while adding thereto intermittently a slime control agent containing as the main component DBNPA (dibromonitrilopropionamide) three times a day each for a duration of 15 minutes at a contact concentration of 2.5 mg/liter. After operation of the test apparatus for 12 days, a slime was formed in a layer thickness of 119 $\mu$m, which was collected and scattered over a PY agar medium (containing 1 g/liter of polypeptone, 1 g/liter of yeast extract, 0.5 g/liter of NaCl and 1.5 % of agar with pH of 7.0) and was cultured thereon, whereupon 24 strains were isolated at random from the maximum dilution agar plate. Each isolated strain was taken up on a platinum loop and was suspended in 0.4 ml of TE buffer solution (10 mM Tris-HCl, 1 mM EDTA, pH 8.0) retained allotted in a 1.5 ml microcentrifugal tube.

To this suspension, there was added 0.8 grams of zirconia/silica beads (supplied from Bio Spec Products, Inc.) of 0.1 mm size and the mixture was homogenized using a cell crusher (BeatBeater of Bio Spec Products, Inc.) at the maximum output power for one minute. After centrifugation at 8,000 G for 5 minutes, the supernatant was used as template DNA solution in a polymerase chain reaction (PCR). For the reagent, PyroBest DNA Polymerase (trademark, Takara Shuzo Co., Ltd.) was used and for the reactor, GeneAmp 2400 (trademark, PE Biosystems Japan, Ltd.) was employed. For the primer, 5'-GAGTTTGATCMTGGCTCAG-3' and 5'-ACGGYTACCTTGTTACGACTT-3' were employd. The PCR was performed for 30 cycles under a reaction condition of 94 ˚C for 0.2 minute, 55 ˚C for 0.3 minute and 72 ˚C for 2 minutes, followed by the last cycle of 72 ˚C for 7 minutes. The resulting reaction product mixture was passed through a MicroSpin S-300 HR column (trademark, Amersham Pharmacia Biotech Corp.) to remove unreacted primer, whereupon a part thereof was subjected to a sequencing reaction using BigDye Terminator Cycle Sequencing FS Ready Reaction Kit (trademark, PE Biosystems Japan, Ltd.). For the primer for sequencing reaction, 5'-GAGTTTGATCMTGGCTCAG-3' and 5'-CAGCMGCCGCGGTAATWC-3' were employed.

The reaction product mixture was passed through AutoSeq G-50 column (trademark, Amersham Pharmacia Biotech Corp.) to remove the unreacted nucleotides, whereupon it was heated to cause denature, before it was passed to ABI PRISM 3000 Sequencer, in order to determine the base sequence for the region of 500 bases from the 5' terminal. By identifying each individual bacterium by making access to GenBank Database with respect to the base sequence data obtained for all the isolated strains, it was confirmed that the isolated 24 strains included 5 kinds of bacteria, among which a bacterium having a 16S rDNA having a homology of 98.1 % with the base sequence of one kind of bacterium (which was identified as Microbacterium sp.) belonging to the genus Microbacterium registered in the DataBase with an accession number AB004728 occupied 75 % of the entire bacteria population, constituting, therefore, the dominant microbe.

Further, by making search in a data base for search for microbe constructed privately (a data base constructed by accumulating privately the results obtained from searches made in the past) as to the base sequence of this isolated dominant microbe, it was confirmed that a bacterium with completely identical base sequence had once been isolated, which was found registered therein with a microbe number 6354. By making search in the data base for search for antimicrobial agent using this microbe number as the search key, it was confirmed that the above Microbacterium sp. is a chemical-resistant bacterium having resistance to DBNPA and that all the other isolated bacteria are susceptible to DBNPA. For the sake of confirmation, sterilizing experiment by DBNPA was carried out for all the isolated bactrinm strains afterwards, which showed that the results obtained from the data base gave correct values.

For the Microbacterium sp., search was made for the antimicrobial agent which brings about antimicrobial effect at the lowest cost performance judged from the value

$$[(\texttt{minimum effective concentration}) \times (\texttt{price})]$$

in the data base for search for antimicrobial agent. By this search, an antimicrobial agent HPGHC, i.e. 2-(p-hydroxyphenyl) glyoxylohydroximoyl chloride, was detected, wherein the minimum effective concentration thereof was determined to be 0.2 mg/liter. All other isolated bacterium strains were susceptible to HPGHC.

Based on the above results, test experiment was carried out under the same experimental condition except that HPGHC was used as the slime control agent so as to maintain the concentration thereof in the system at 0.3 mg/liter. By this experiment, it was recognized that the amout of cling substance was remarkably decreased and that the slime had grown in the same test period only to a thickness of 3 $\mu$m. This HPGHC-treated slime collected at this occasion was tested by analysis of microbial biota thereof in the same manner as above, whereby Microbacterium sp. was not detected.

In this Example, the time interval required after the sampling of the slime till confirmation that the Microbacterium sp. was the dominant microbe was 5 days.

The data base for search for antimicrobial agent employed in Example 1 was that constructed by making use of Microsoft Access (trademark, Microsft Corp.), by inputting therein the results of sterilizing experiments and growth inhibiting experiments carried out for bacteria isolated from each of water systems and from the starch slurry, starch paste and coating color liquor of a paper manufacturing factory using various antimicrobial agents (slime control agents, preservatives and the original components of them) under conditions of three levels of pH and three levels of temperature and by inputting further the data integrally gathered from the practical results in the past obtained using various antimicrobial agents.

The search in the data base for search for antimicrobial agent was performed using only the functions of "sort", "autofilter" and "search" installed originally in the Microsoft Access, wherein judgement was made by displaying the result of search on a computer display.

EXAMPLE 2

<<Example of Selection of Slime Control Agent at Paper Manufacturing Factory D>>

On the course of slime control which as been continued over a long term using an antimicrobial agent constituted mainly of DBNPA in a Foudrinier machine operating for papermaking of medium quality paper, occurrence of a pink-colored bacterial slime was recognized in the white water circulation system. Therefore, procedures corresponding to those in Example 1 were proceeded, wherein each 24 cell strains were isolated from each microbe in the slime collected at each of the locations where slime was found, whereupon each strain was cultured on an agar medium to attain analysis of the microbial biota of the collected slime. The results were such that the isolated cell strains included 5 species of bacteria, wherein a bacterium which was found to have a 16S rDNA having a homology of 99.5 % with Deinococcus geothermalis recorded in the data base of GenBank with an accession number AJ000002 (and which was therefore identified as D. geothermalis) was found as occupying 46 % of the entire microbe population.

By making search in the same data base for search for antimicrobial agent as in Example 1 using Deinococcus geothermalis as the search key, it was found that this bacterium belongs to a group resistant to DBNPA. For the sake of confirmation, a sterilizing experiment for examining resistance of this microbe to DBNPA was carried out afterwards, which showed that this bacterium belongs certainly to a bacteria group resistant to DBNPA. By picking up antimicrobial agents effective for this bacterium from the data base for search for antimicrobial agent, it was recognized that a certain inorganic antimicrobial agent based on bromide is adaptive under the condition of temperature and pH in this system. From a further search made for other isolated microbes in this slime, it was found that there was no microbe constituting this slime exhibiting a resistance to this inorganic antimicrobial agent based on bromide. Therefore, this anitimicrobial agent was applied for actual slime control treament at once, which resulted in that occurrence of not only the pink-colored slime but also all other slimes was reduced remarkably. When a sample of adherent materials formed in the white water silo was collected after a continuous paper manufacturing operation for 25 days and the analysis of the microbial biota thereof was performed in the same manner as above, it was found that the occupation proportion of Deinococcus geothermalis in the entire microbial biota was reduced to 4 %. Here, the time required after the sampling of the slime until attainment of decision of new treating method via the termination of the experiment was 6 days.

## EXAMPLE 3

<<Example of Monitoring at Paper Manufacuring Factory D>>

In the paper manufacturing factory D, another machine of a type similar to that used in Example 2 operating under conditions similar to those in Example 2 is installed adjacent to the machine of Example 2. In this machine, no occurrence of pink-colored silme was recognized even at the occasion at which the pink-colored slime occurred in the machine of Example 2. Therefore, procedures corresponding to those in Example 1 were proceeded, wherein each 36 strains were isolated using agar medium and analysis of the microbial biota was carried out. Since a large number of strains are to be tested, each 16S rDNA obtained from each strain was treated by digestion with three kinds of restriction enzymes (BstUI, RsaI and HhaI) isolatedly to effect TRFLP analysis, in order to group preliminarily into classes of microbes of same reaction pattern, whereupon identification was effected based on the base sequence as identification parameter. It was recognized thereby that the occupation proportion of Deinococcus geothermalis in the isolated 36 strains was calculated to be only2.7 % (one strain).

Since the machine construction is similar, this machine would suffer from same trouble by occurrence of pink-colored slime after a prolonged operation. Therefore, monitoring of machine by prior practice was proceeded more enhancedly and, at the same time, the' analysis of the microbial biota of white water same as in the above test method was performed at a more frequent interval, namely, at an interval of 7 - 14 days (with increase in the number of isolated strains to 48). In addition, aligned three monitoring demonstrators of basically the same construction as that of the slime tester of Example 1 were installed in this machine and were operated in the manner as follows:

One demonstrator was used as an intrinsic demonstrator (provisionally named No. 1 demonstrator) and was supplied continuously with the white water from the machine. The two remainders were used each as a simulator demonstrator operated to simulate the operation condition of the paper machine, wherein the feed rate of the white water to the simulators was adjusted so as to settle the residence time therein at the same value as that of the paper machine. On the other hand, white water devoid of DBNPA content was reserved by storing in a storage reservoir the white water taken from its supply line during a time interval discrepant from the DBNPA injection period, to preserve a reagent-free white water for the test. Using each independent chemical injection system, one simulator (denoted as No. 2 demonstrator) was supplied with a DBNPA-containing tereating agent and the other one (denoted as No. 3 demonstroator) was supplied with an an inorganic bromine compound each under the condition same as that of the machine so as to simulate the treating condition of the machine, in order to examine the antimicrobial effect of the DBNPA-containing agent and that of the inorganic bromine compound under the condition same as the machine.

The results of analyses of the microbial biota were within the range of the occupation proportion of Deinococcus geothermal is of 2 - 4 % (1 or 2 strains in the 48 strains) up to the fourth test cycles. This proportion rised to 6 strains among the entire strains (12.5 %) in the analysis result obtained in the fifth test cycle performed on the 47th day after the first test. In the concurrently performed tests for the effect of antimicrobial agents for white water, the effect of DBNPA-containing treating agent was better within te range of usual fluctuation up to that day. On the other hand, in the No. 3 demonstrator in the aligned three demonstrators, no sign of growth of slime was noted. In No. 1 and No. 2 demostrators, a sign of growth of slime was recognized, though adherent deposit of slime was only within a slight extent of a slime thickness of a few $\mu$ m.

By integrating these plurality of results, it was judged that the above change in the analysis result was a sign of beginning of formation of the pink-colored slime also in the paper manufacturing machine. Therefore, the antimicrobial agent applied to the machine was changed from the DBNPA-containing agent to the inorganic bromine compound (on the 5th day from the start of the test).

The result of analysis of the microbial biota of white water of the machine performed on the 7th day after the change of the amtimicrobial agent showed that the original state of biota was almost regained, as the microbe Deinococcus geothermalis was present in only one strain among the isolated 48 strains (occupation proportion of 2 %). The amount of slime deposition in the No. 1 demonstrator among the aligned three demonstrators was below the lowest detectable limit. In contrast thereto, the deposited amount of slime in the No. 2 demonstrator increased gradually and occurrence of the pink-colored slime was visually recognized on the 14th day after the change of the antimicrobial agent. From this, it was judged that this machine would have, at high probability, suffered from slime trouble if the change of antimicrobial agent was delayed for a couple of weeks.

From the above, it is made clear that the method for monitoring antimicrobial effect according to the present invention is useful as a means for monitoring the effectiveness of the antimicrobial treatment in a paper manufacturing machine held under treatment with antimicrobial agent(s).

EXAMPLE 4

<<Example of Selection of Slime Control Agent at Paper Manufacturing Factory 0>>

On the course of slime control which had been continued over a long term by a cocurrent application of a chlorine treatment and an antimicrobial agent constituted mainly of DBNPA in a Foudrinier machine operating for papermaking of medium quality neutral paper, occurrence of a yellow-colored slime was recognized in the white water circulation system. Therefore, in the same manner as in Example 1, each 24 strains were isolated from the slime and analysis of the microbial biota by 16S rDNA was effected. It was made clear thereby that 90 % of the microbial biota were occupied by a microbe having a gene that has a homology of 98.0 % with the base sequence of 16S rDNA of a microbe belonging to a genus Microbacterium to be identified from the data base of GenBank with an accession number AB027702.

Then, by search in the above-mentioned privately constructed data base for search for microbe as to the base sequence of the isolated dominant microbe, it was found that a microbe having a base sequence which has a homology of 99.1 % with that of the isolated dominant microbe had been registered therein with a microbe number 2401. Therefore, search was made in a data base for search for antimicrobial agent using this microbe number as the search key, whereby it was confirmed that this dominant microbe belongs to a microbe group exhibiting resistance to DBNPA. Judgement based on the value

$$\mathtt{[(minimum\ effective\ concentration)\times\ (price)]}$$

under the environmental condition of the machine indicated that an antimicrobial agent exhibiting efffectiveness on this dominant microbe is an antimicrobial agent containing, as the main reagent, an inorganic bromine compound. Therefore, the slime control treatment was performed from the subsequent operation on using solely an antimicrobial agent based on inorganic bromine compound that was selected. Thereafter, during the continued operation for 14 days, no visually discriminable occurrence of slime was detected.

EXAMPLE 5

<<Example of Selection of Preservative for Starch Slurry at Paper Manufacturing Factory A>>

In a starch paste (pH 9, 60 ˚C) for papermaking at the paper manufacturing factory A, a phenomenon occurred in which the viscosity thereof had decreased within a short time. In this system, an antimicrobial agent based on isothiazolone was used by injecting continuously into the system at a concentration in terms of isothiazolone concentration of 10 mg/l. Observation of microbe cell count showed a population of $(1.7 \times 10E+ 8CFU)$/ml, which indicated an early stage of rotting. By performing the analysis of the microbial biota in the manner as in Example 3, it was confirmed that 96 % of the entire microbial biota were occupied by a microbe having a 16S rDNA having a homology of 94.8 % with that of a microbe which belongs to the genus Paenibacillus (GenBank data base; AJ288158).

Then, by search in the above-mentioned privately constructed data base for search for microbe as to the base sequence of the isolated dominant microbe, it was found that a microbe having completely identical base sequence had been registered therein with a microbe number 2675. Therefore, search was made in a data base for search for antimicrobial agent using this microbe number as the search key, whereby it was made clear that this dominant microbe had once been isolated in the same paper manufacturing factory and that the vegetative cell for this microbe is susceptible to isothiazolone. Since the dominant microbe is reagent-susceptible, the cause of the above phenomenon was assumed to be not based on inadaptability of the selected antimicrobial agent but based on the manner of application of the antimicrobial agent. Therefore, re-inspection was made for the antimicrobial agent injection system, whereby it was found that the delivery rate of the chemical injection pump was reduced to about 1/5 of the nominal rate, resulting in insufficient application concentration. The reduction of the rate of injection was supported also from the amount of the preservative solution remained in the preservative storage. Thus, based on this judgement, cleaning of the starch paste storage was performed and the chemical injection pump was replaced by a new one, whereupon the operation was started again. The above phenomenon was removed and proper microbe cell count was regained.

EXAMPLE 6

<<Example of Selection of Slime Control Agent for Filamentous Fungi at Paper Manufacturing Factory S>>

On the course of slime control which had been continued over a long term using an antimicrobial agent containing as main reagents DBNPA and BBAB (1,4-bis-(bromoacetoxy)-2-butene) in a Foudrinier machine operating for papermaking of news printing acidic paper, slimes mainly of fungi occurred over the entire machine. Therefore; selective separation of filamentous fungi and yeast was performed using PYD meduim. The colonies formed after culture for 4 days were all constituted of filamentous fungi in grains of a diameter of 5 mm with slightly orange-colored hyphae. Eight strains were isolated in the same manner as in Example 1 and were subjected to analysis of microbial biota by 18S rDNA. For the PCR primer and for the sequencing primer, there were employed 5'-GTAGTCATATGCTTGTCTC-3' and 5'- GGCTGCTGGCACCAGACTTGC-3', respectively. It was recognized thereby that all the 8 strains are constituted of a filamentous fungus having an 18S rDNA which has a homology of 99.2 % with Chaetomium elatum (GenBank data base; accession number M83257).
By search in a data base for search for antimicrobial agent using Chaetomium elatum as the search key, it was confirmed that this filamentous fungus belongs to a group of filamentous fungi non-susceptible to DBNPA and BBAB. Judgement based on the value

$$[(\text{Minimum effective concentration}) \times (\text{price})]$$

under the environmental condition of the machine indicated that an antimicrobial agent exhibiting effectiveness on this filamentous fungus is an antimicrobial agent containing, as the main component, 4,5-dichloro-1,2-dithiolan-3-one (in the following, called the dithiol). Therefore, the slime control treatment was performed from the subsequent operation on using an antimicrobial agent based on BBAB and the dithiol that were selected. Thereafter, during the continued operation for 14 days, no visually discriminable occurrence of slime was detected.

EXAMPLE 7

<<Example of Selection of Slime Control Agent in Cooling Water System for Air Conditioner>>

In a cooling water system for air conditioner in the administration block of Kabushiki Kaisha N in which slime control treatment was kept by immersing a solid tabletted antimicrobial agent, prepared by compacting under clathrated isothiazolone into tablet, in the cooling water, a significant outbreak of microbial slime occurred. Since the system uses cooling water with scarce content of organic substance, it was presumed that the microbial biota therein should be composed largely of non-culturable microbes. Therefore, it was decided to take steps of extracting entire DNA from the slime and performing PCR using the so-extracted entire DNA as the template.
Using a 2 ml capacity microcentrifugal tube equipped with a screw cap, a spaturaful slime was suspended in 1 milliliter of a DNA extracting buffer solution (100 mM Tris-HCl, 100 mM EDTA, 100 mM $Na_2 HPO_4$ + $NaH_aPO_4$ and 1.5 M NaCl; pH 8.0) and the suspension was homogenized after admixing thereto 2 grams of 0.1 mm zirconia/silica beads using a beatbeater at its maximum output power for two minutes. The suspension was further processed by three repeated freezing/thawing cycles, whereupon the mixture was subjected to reaction by adding thereto 0.01 ml of Proteinase K (of Seikagaku Kogyo Co., Ltd.) of a concentration of 10 mg/ml for a reaction time of 15 minutes at 37˚C. Thereto was further added 0.2 ml of 10 % SDS with agitation, whereupon the reaction mixture was stood still for 30 minutes at 65 °C.

After homogenization again using the beatbeater, the resulting mixture was subjected to centrifugation (10,000 G, 10 minutes), whereupon the supernatant was collected. An equal volume of chloroform was added thereto and the mixture was shaken suffuciently, before it was centrifugalized (10,000 G, 10 minutes), whereupon the supernatant was recovered. Thereto was added a 0.6 time volume of isopropanol with agitation, whereupon it was stood still at room temperature for 30 mintes, followed by centrifugation (10,000 G, 10 minutes) to recover the DNA. The collected DNA was rinsed with 70 % ethanol and was dried, before being suspended in TE buffer solution. The PCR was carried out in the same manner as in Example 1. As the primer, there were used 5'-GAGTTTGATCMTGGCTCAG-3' and 5'-CAGCMGCCGCGGTAATWC-3'. For the latter, that modified by phosphatization at the 5'-terminal was employed and about 500 base sequences were amplified. The resulting PCR product was treated by $\lambda$-exonuclease into single stranded DNA to realize analysis by SSCP method. Thus, an electrophoresis was performed using a 10 % polyacrylamide gel at 200 volt under a low temperature condition of 20 °C or a period of time of 8 hours. After the gel was stained with GelStar (trademark, of Takara Shuzo Co., Ltd.), DNA bands were confirmed by irradiation by ultraviolet lamp thereonto. About 10 independent bands were recognized, though bands for considerably high concentrations (that is, greater contents) were not detected.

For a slime in this cooling water system which had been collected unintentionally and held stored under frozen state, similar analysis was performed. By comparison of the result of this analysis with the result of the afore-mentioned analysis, it was recognized that no significant discrepancy in the number of bands and in the position and intensity of corresponding band was found between them, indicating that no variation in the microbial biota happened before and after the occurrence of the slime trouble. From the band of the highest concentration among the detected bands, DNA was collected and the base sequence thereof was determined. It was recognized that the DNA in the band is composed of a 16S rDNA which has a homology of 95.8 % with that of a microbe closely related to genus Rubrivivax (data base of GenBank; accession number X89910) and 16S rDNA which has a homology of 99.5 % with that of Sphingomonas sp. (data base of GenBank; accession number AB023290).

By search in the same privately constructed data base for search for microbe as in Example 1, it was found that these 16S rDNA had often been isolated in the past from this cooling water system without any record of significant slime occurrence by treating with isothiazolone. By research also in the same data base for search for antimicrobial agent as in Example 1, it was noted that Sphingomonas sp. is a microbe susceptible to the antimicrobial agent. From all these, it was assumed that it would be probable that the trouble was not due to inadaptability of the antimicrobial agent now on use but due to a problematic manner of application thereof. Therefore, inspection was made into the daily operation report, which showed that an alteration of operation scheme was entered by the operation personnel by increasing the water below out level, whereby the residence time of the cooling water in the cooling water system is reduced, resulting in a lowering of the concentration of isothiazolone in the water to a level permitting growth of slime. By regaining the rate of water blow out to the rated revel, occurrence of slime was ceased.

EXAMPLE 8.

<<Example of Antimicrobial Treatment on the Side of RO Concentrate in Ultrahigh Purity Water Production>>

On the side of RO concentrate in a ultrahigh purity water production line at an electronics manufacturing factory H, a slime trouble occred, which caused a trend to reduction of flux. The slime was suspended in a DNA extracting buffer solution and was subjected to homogenization after addition thereto glass beads. Thereafter, the procedures of Example 7 were pursued to effect extraction of DNA, whereupon the 16S rDNA fragments for all the extracted DNA strands were prepared. After treated into single strand, analysis was performed by SSCP method. Five independent bands were recognized, of which one was found as occupying 80 % of the integral intensity. By determining the base sequence for this band, it was found that the microbe has a homology of 99.9 % with Ralstonia eutropha (GenBank; accession number D88000).

A plurality of Ralstonia eutropha are registered in the data base for search for antimicrobial agent used in Example 1, which have, by comparaison with the 16S rDNA isolated above, each a homology of 99.2 - 99.7 % with it. By search, sodium hypochlorite was recognized as the most effective antimicrobial agent for all of them. However, use thereof is prohibited, since it has been known that chlorine acts on the RO membrane damagingly. Therefore, further search was made for an antimicrobial agent which is not damaging to the RO membrane but is effective for the above-mentioned microbes in the data base for search for antimicrobial agent, whereby it was found that an antimicrobial agent based on isothiazolone had recorded an effective experience in a practical application when it was used at a dosage of 1.2 - 1.5 mg/$\ell$ as converted to isothiazolone. Accordingly, the so retrieved antimicrobial agent based on isothiazolone was introduced into the RO concentrate side space at a dose rate of 1.5 mg/$\ell$, after cleaning there. No slime formation was recognized any more.

REFERENCE EXAMPLE 1

In a process course of papermaking for neutral paper, slime formation occurred in the white water circulation system of paper machine after a continuous operation for about two weeks. A slime control treatment was applied thereto by a method of introducing a slime control agent containing DBNPA (dibromonitrilopropionamide) as the effective component into the stuff box intermittently (by an automatic introduction of a treating liquid dose, settled so as to maintain the DBNPA concentration in the system at 20 mg/$\ell$ for 20 minutes, by a metering pump four times a day). In this paper manufacturing factory, a further slime formation trouble occurred also in the clear white water line, wherefor another slime control treatment was performed by introducing a slime control agent containing also DBNPA as the effective component into the clear white water pit intermittently.

EXAMPLE 9

After two months from the start of the treatment given in the Reference Example 1, occurrence of slime constituted mainly of bacteria was recognized in the suction box beneath the wire section of the paper machine. From a sample of this slime, microbes were cultured by isolation culture on agar plate. For the scooped 48 strains, analysis of the microbial biota was performed using 16S rDNA as the analysis parameter. Each 16S rDNA was synthesized by PCR using the DNA extracted from each of the strains as the template, whereupon it was subjected to digestion using three restriction enzymes (BstUI, Rsa1 and HhaI) to effect RFLP analysis. A preliminary grouping was made judging that microbes showing electrophoresis pattern in accord with each other belong to identical microbe, whereupon each microbe was identified by search in the data base of GenBank by access thereinto using the base sequence at the portion around the site of 500 bp from the 5'-terminal as a search parameter. It was confirmed thereby that, among the 48 strains, 25 strains are Deincoccus geothermalis, 10 strains are Acidovorax temperans, 8 strains are a Gram-negative bacterium near-related to the genus Riemerella, 4 strains are Sphingomonas and the last one strain is Rhizobium sp.

By search in the data base for search for antimicrobial agent using the base sequence of 16S rDNA for each microbe as a search parameter, it was recognized that Acidovorax temperans, the Gram-negative bacterium near-related to Riemerella, Shingomonas and Rhizobium sp. are susceptible to DBNPA, whereas Deinococcus geothermalis is resistant to DBNPA.

In order to confirm this, a sterilizing experiment was carried out for a representative isolated strain afterwards, which showed that 99 % of living microbe cells had become extinct for Acidovorax temperans, a Gram-negative bacterium near-related to Riemerella, Sphingomonas and Rhizobium sp. after contact with DBNPA at a concentration of 2 mg/$\ell$ or even lower for a contact time of 30 minutes, whereas Deinococcus geothermalis did completely not sterilized by DBNPA even by contact therewith at 30 mg/$\ell$ for 30 minutes.

Samples were collected at various locations in the paper manufacturing process courses and, for each of them, total microbe cell count was measured by microscope direct count method after staining with DAPI, whereupon 48 strains were isolated by agar plate culture to determine the proportion of existence of Deinococcus geothermalis among the 48 strains based on the base sequence of 16S rDNA. The results of the experiment are recited in Table 1.

Table 1

| Sample or sampling location | Total cell count (cells/ml) | Number of strains of Deinoc. geotherm. (Numb. of colonies among 48 strains) | Proportion (%) *1 |
|---|---|---|---|
| White water in white water silo | $5.1 \times 10^7$ | 9 | 19 |
| Clear white water in clear white water pit | $3.0 \times 10^7$ | 8 | 17 |
| Industrial water | $5.7 \times 10^8$ | 0 | 0 |
| LBKP chest | $6.0 \times 10^6$ | 1 | 2 |
| CGP chest | $4.8 \times 10^4$ | 0 | 0 |
| Dry broke chest | $8.3 \times 10^7$ | 7 | 15 |
| Wet broke chest | $6.7 \times 10^7$ | 44 | 92 |
| Mixing chest | $4.8 \times 10^7$ | 6 | 13 |
| Machine chest | $5.2 \times 10^7$ | 7 | 15 |
| Dyestuff tank | $1.1 \times 10^3$ | 0 | 0 |
| Sizing agent tank | $8.7 \times 10^2$ | 0 | 0 |
| Ext. starch paste tank | $1.5 \times 10^4$ | 0 | 0 |

(continued)

| Sample or sampling location | Total cell count (cells/ml) | Number of strains of Deinoc. geotherm. (Numb. of colonies among 48 strains) | Proportion (%) *1 |
|---|---|---|---|
| Int. starch paste tank | $4.6 \times 10^8$ | 0 | 0 |
| [(number of colonies of Deinococcus geothermalis) /48] × 100 <br> Note: *1: | | | |

Deinococcus geothermalis was present mainly in the wet broke chest at an existence proportion which reached 91.7 %. While this microbe was found also in the LBKP chest and dry broke chest, they were brought from the recovered white water used for preparation of pulp. It is seen that Deinococcus geothermalis did not grow in the mixing chest and in machine chest, where pulp raw materials gather, since the occupation proportion of Deinococcus geothermalis therein was low notwithstanding the total cell count increased there. From the results of the experiment given above, it was concluded that Deinococcus geothermalis, which is resistant to DBNPA, proliferated in the wet broke chest and was brought therefrom to the white water circulation system.

By making search in a data base for search for antimicrobial agent using Deinococcus geothermalis as the search key and confining the extent of retrieval into slime control agents having high antimicrobial activity in neutral range, HPGHC [2-(p-hydroxyphenyl)-glyoxylohydroximoyl chloride] was picked up. It was also recognized that Deinococcus geothermalis is sterilized by contacting with HPGHC at a system internal concentration of 20 mg/ℓ for 20 minutes. Since the flow amount through the wet broke system is about 1/20 of that flown into the white water circulation system, amount of consumption of the chemical can be economized by introducing it into the wet broke system, in order to attain the system internal concentration of 20 mg/ℓ. Moreover, it may bring about an advantage of permitting to spare the amount of chemical to be used as compared with that calculated from the flow amount ratio, since the state of contact of microbes with the chemical can be kept over a prolonged time interval without having recourse to continuous addition of the chemical due to the larger capacity of the wet broke chest permitting longer residence time of the chemical therein to maintain the microbes held under contact with the chemical for longer time.

The data base for search for antimicrobial agent used in this Example had been constructed by making use of Microsoft Access (trademark) of Microsoft Corp., wherein preservative experiments and growth inhibiting experiments were performed using various slime control agents or the original components thereof under experiment conditions at three pH levels and three temperature levels for microbes isolated from various water systems and from starch slurry, starch paste and coating color liquor in paper manufacturing factories, whereupon the results of these experiments were inputted therein together with the integral data that were obtained in the past from practical treatment works with slime control agents applied to various objective systems. The search in this data base was made by utilizing only the functions "sort", "autofilter" and "search" built in originally in Microsoft Access and the search result was displayed on an image screen of a computer to decide the judgement.

Based on the results of the search for slime conrol agent, the treatment scheme was revised to the operation in which a slime control agent containing HPGHC as the effective component is added as the origin treating agent to the wet broke chest three times a day each at a dose rate of 25 milligrams per liter of the holding volume (chest capacity), while supplying the white water circulation system with a slime control agent containing DBNPA as the effective component in the conventional manner.

The amount of HPGHC used here corresponds to about 1/30 of that in which the chemical is assumed to be added to the white water circulation system.

As a result, the amount of slime formation was reduced considerably as compared with that observed upon shut down of machine operation and the cling substance slightly found in the suction box was changed in the constituent from microbial slime to suspended matter mainly of pulp fibers.

At the occasion of the second shut down after the revision of the treatment scheme and at an occasion directly before it, samples were collected at various locations in the paper manufacturing process courses, for which total microbe cell count and the proportion of existence of Deinococcus geothermalis were determined in the same way as the former practice. It was found thereby that the total microbe counts were unchanged and within the range of ordinary fluctuation, but Deinococcus geothermalis became undetectable. The results are recited in Table 2.

Table 2

| Sample or sampling location | Total cell count (cells/ml) | Number of strains of Deinococcus geothermalis (number of colonies among 48 strains) |
|---|---|---|
| White water in white water silo | $4.8\times 10^7$ | 0 |
| Clear white water in clear white water pit | $3.1\times 10^7$ | 0 |
| Dry broke chest | $6.0\times 10^7$ | 0 |
| Wet broke chest | $5.3\times 10^7$ | 0 |

Based on the results as above, it is seen that Deinococcus geothermalis, which is resistant to DBNPA, was exterminated at the site of origin of occurrence thereon, namely, the wet broke chest, by only an additional application of small amount of an additional slime control agent. It is also seen that the effect of slime control in the white water circulation system had been assured.

By the way, the total microbe cell count in the sample of cling substance collected at the same time from the suction box was determined to be $3.5 \times 10^9$ cells per one gram of dry substance, wherein there was no Deinococcus geothermalis detected therefrom.

EXAMPLE 10

In a paper machine Z (a daily throughput of 290 tons, high grade paper, pH 7.3) of a paper manufacturing factory A, occurrence of brown defects having sized of 1 - 1.5 mm was recognized frequently since directly after the start of operation. The defect portions were cut out from the paper and about 30 cut out pieces (about 7.5 mg) were placed in a 2 ml microcentrifugal tube made of plastic material. To this tube, 1 ml of a DNA-extracting buffer solution (100 mM Tris-Cl, 100 mM EDTA-Na, 100 mM $Na_2HPO_4$ and 1.5 M NaCl (pH 8.0)) was added, followed by standing still at room temperature for 30 minutes to cause the paper sheets to be impregnated sufficiently with the solution.

After addition of 2 g of 0.1 mm zirconia/silica beads (BioSpec Products, Inc.) thereto, the mixture was homogenized using a cell crusher (Beatbeater, of BioSpec Products, Inc.) for 2 minutes. Then, 10 $\mu$ l of an aqueous solution of PROTEINASE K (of Seikagaku Kogyo Co., Ltd.) of a concentration of 10 mg/ml were added thereto and the reaction was effected at 37 ·C for 15 minutes. Thereto were added then 250 $\mu$ l of 10 % SDS aqueous solution and the mixture was subjected to homogenization using Beatbeater for 1 minute, followed by standing still at 60 ·C for 30 minutes. After centrifugation at 12,000 rpm at 25˚C for 10 minutes, 600 $\mu$ l of the supernatant were transferred to a new plastic tube and thereto were added 600 $\mu$ l of chloroform. After a sufficient agitation, the mixture was subjected to centrifugation at 12,000 rpm at 25˚C for 10 minutes. Then, 550 $\mu$ l of the supernatant were transferred to a new tube, 330 $\mu$ l of isopropanol were added thereto and the mixture was agitated mildly, before being stood still at room temperature for 30 minutes. After centrifugation (12,000 rpm, 25 ˚C, 10 minutes), the supernatant was discarded and the tube inside was rinsed with 70 % ethanol, whereupon the precipitate was dried under a reduced pressure. The dried precipitate was then suspended in 50 $\mu$ l of an aqueous TE solution (10 mM Tris-Cl, 1 mM EDTA (pH 8.0)) to prepare a DNA suspension. For the normal portion of the paper also, a part of the paper corresponding to 7.5 mg was cut equally into 30 pieces, for which the above procedures were pursued to effect extraction of DNA. For the sake of confirmation of the reproducibility, the procedures for the extraction were performed in double series for each sample.

Then, using each of the suspensions of DNA that were extracted from the defect portion and from the normal portion by the above procedures as the template, amplification of s s rDNA was carried out by PCR using bacteriospecific primers (Bact27f: 5'-AGAGTTTGATCMTGG-CTCAG-3', Bact519R: 5'-GWATTACCGCGGCKGCTG-3') and fungalspecific primers (NS1: 5'-GTAGTCATATGCTTGTCTC-3', NS2: 5'-GGCTGCTGGCACCAGACTTGC-3'). As the reagent, PyroBest DNA polymerase (of Takara Shuzo Co., Ltd.) was used and, as the reactor, GeneAmp 2400 (of Applied Biosystems Japan, Ltd.) was employed. PCR was carried out in a reaction liquid of 30 $\mu$l in 30 repetition cycles each under a condition of 94˚C for 0.2 minute, 55 ˚C for 0.3 minute and 72 ˚C for 1 minute, with final cycle of 72˚C for 7 minutes.

2 $\mu$ l of the amplification product were subjected to an electrophoresis on 2 % agarose gel at 100 mV for 45 minutes, whereupon staining of DNA was effected by ethidium bromide staining, in order to observe under UV ray irradiation. The observation showed that bands for the s s rDNA were seen both for the defect portion and normal portion when the bacteriospecific primer was used, whereas, when the fungalspecific primer was used, band for 18S rDNA was recognized only for the defecct portion but not for the normal portion. From this, it was recognized that the microbe amount in the defect portion was in the same level as that in the normal portion and that the defect portion contained a marked proportion of fungi.

25 $\mu$ l of aqueous solution of the amplified 18S rDNA were passed to MicroSpin S-300 HR column (of Amersham Pharmacia Biotech Corp.), followed by removal of unreacted primer, whereupon a part of the reaction product was subjected to sequencing reaction using BigDye Cycle Sequencing FS Ready Reaction Kit (of Applied Biosystems Japan, Ltd.). For the sequencing primer, the above-mentioned NS 1 and NS 2 were used and, for sequencer, ABI Prism 310 Genetic Analyzer (of Applied Biosystems Japan, Ltd.) was employed. There was obtained the base sequence designated by Sequence No. 6 in the Sequence Table.

Search was carried out for the base sequence of Sequnce No. 6 in a data base. Thus, by access to the data base of GenBank to compare with the 18S rDNA sequences stored in the data base using BLAST, it was recognized that base sequence of Sequence No. 6 coincides at the most with the 18S rDNA sequence of filamentous fumgi of genus Sarcinomyces and has a homology of 94 % therewith.

The site of origin of occurrence of the filamentous fungus belonging to genus Sarcinomyces identified in the manner as above was determined by the procedures as given below. Thus, colony count of this filamentus fungus was determined for the white water and for the lines for the raw materials based on the number of colonies formed on PDA plate medium as the parameter of the count. Further, identification of each fungus was carried out by the DNA sequencing as above using the base sequence of 18S rDNA of the colony as the identification parameter, after the colonies had been subdivided into groupes according to the form of colony as the discrimination parameter. It was found thereby that the base sequence of the DNA of the red brown mold recognized as the dominant microbe, as seen in Table 3, in the white water and in the LBKP slurry coincides completely with the base sequence of DNA of fungus isolated from the defect portion.

From the above, it was made clear that the defect was caused by the red brown mold and that many of such molds were brought from the LBKP slurry.

Table 3 Count of Fungi (CFU/ml)

|  | White water | Stuff box slurry | LBKP slurry | NBKP slurry | Broke slurry |
|---|---|---|---|---|---|
| Total microbe count | $2.1 \times 10^1$ | $4.1 \times 10^1$ | $1.0 \times 10^2$ | not detected | $4.0 \times 10^\circ$ |
| Red brown, 2-3 mm felt-like hypha | $1.9 \times 10^1$ | $3.6 \times 10^1$ | $1.0 \times 10^2$ | not detected | $2.0 \times 10^\circ$ |
| White, 10 - 15 mm, woolly | $1.0 \times 10^\circ$ | $2.0 \times 10^\circ$ | not detected | not detected | not detected |
| White, green center, 15 - 20 mm, woolly | not detected | $1.0 \times 10^\circ$ | not detected | not detected | $1.0 \times 10^\circ$ |
| Others | $1.0 \times 10^\circ$ | $2.0 \times 10^\circ$ | not detected | not detected | $1.0 \times 10^\circ$ |

Based on the above results, countermeasure for preventing occurrence of defect was incorporated in the following manner. Inspection was made first in the LBKP production process course, whereby it was made clear that occurrence of slime of red brown color was recognized in the filtrate line from the thickener. The fungus colony count of this slime was detected to be $5 \times 10^6$ CFU per one gram (wet weight) of the slime. All the detected individuals of the fungus were the same as that of the red brown colored mold of the dominant microbe of the white water and also the coincidence in the base sequence therebetween was confirmed. Also from visual observation under microscope, it was recognized that the slime was composed mainly of mold. Therefore, a sterilization/preservation treatment was performed for the filtrate line, after washing this line using NaOH, wherein the treatment was carried out by introducing intermittently into the line a slime control agent containing 4,5-dichloro-1,2-dithiolan-3-one as the main component, which had been proven that it was at the most effective for the fungi isolated from the slime in the filtrate line. This resulted in complete exclusion of occurrence of defect.

EXAMPLE 11

In a paper machine Y (a daily throughput of 410 tons, slightly coated paper, pH 7.5) of a paper manufacturing factory B, occurrence of light brown-colored defects having sizes of about 5 mm diameter was recognized frequently since 11th day from the start of operation, caused to result in cessation of operation. The defect portions were cut out from the paper and three cut out pieces (about 8.1 mg) were cut into 2 mm squares, which were placed in a 2 ml microcentrifugal tube made of plastic material. To this tube, 1 ml of a DNA-extracting buffer solution (100 mM Tris-Cl, 100 mM EDTA-Na, 100 mM $Na_2HPO_4$ and 1.5 M NaCl (pH 8.0)) was added, followed by standing still at room temperature for 30 minutes to cause the paper pieces to be impregnated sufficiently with the solution.

After addition of 2 g of 0.1 mm zirconia/silica beads thereto, the mixture was homogenized using Beatbeater for 2 minutes. Then, 10 $\mu$l of an aqueous solution of PROTEINASE K of a concentration of 10 mg/ml were added thereto and the reaction was effected at 37 ˚C for 15 minutes. Thereto were added then 250 $\mu$l of 10 % SDS aqueous solution and the mixture was subjected to homogenization using Beatbeater for 1 minute, followed by standing still at 60 ˚C for 30 minutes. After centrifugation at 12,000 rpm at 25˚C for 10 minutes, 600 $\mu$l of the supernatant were transferred to a new plastic tube and thereto were added 600 μl of chloroform. After a sufficient agitation, the mixture was subjected to a centrifugation at 12,000 rpm at 25 °C for 10 minutes. Then, 550 μl of the supernatant were transferred to a new tube, 330μl of isopropanol were added thereto and the mixture was agitated mildly, before being stood still at room temperature for 30 minutes. After centrifugation (12,000 rpm, 25 °C, 10 minutes), the supernatant was discarded and the tube inside was rinsed with 70 % ethanol, whereupon the precipitate was dried under a reduced pressure. The dried precipitate was then suspended in 50 μl of an aqueous TE solution (10 mM Tris-Cl, 1 mM EDTA (pH 8.0)) to prepare a DNA suspension. For the normal portion of the paper also, a part of the paper corresponding to 8.1 mg of such normal paper were cut equally into 25 pieces, for which the above procedures were pursued to effect extraction of DNA. For the sake of confirmation of the reproducibility, the procedures for the extraction were performed in double series for each sample.

Then, using each of the suspensions of DNA that were extracted from the defect portion and from the normal portion by the above procedures as the template, amplification of s s rDNA was carried out by PCR using, as in Example 10, bacteriospecific primers (Bact27f, Bact519R) and fungalspecific primers (NS1, NS2). As the reagent, PyroBest DNA polymerase was used and, as the reactor, GeneAmp 2400 was employed. PCR was carried out in 30 repetition cycles each under a condition of 94 °C for 0.2 minute, 55 °C for 0.3 minute and 72°C for 1 minute, with final cycle of 72°C for 7 minutes.

2 μl of the amplification product were subjected to an electrophoresis on 2 % agarose gel at 100 mV for 45 minutes, whereupon staining of DNA was effected by ethidium bromide staining, in order to observe under UV ray irradiation. The observation showed that clear bands for the s s rDNA were recognized for the defect portion while only considerably weak bands were seen for the normal portion when the bacteriospecific primer was used, whereas, when the fungalspecific primer was used, no band was recognized both for the defecct portion and for the normal portion.

From this, it was seen that the defect portion contained a remarkable amount of microbes as compared with the normal portion.

During the period in which occurrence of defect appeared frequently, the system inside was so polluted wholly that detection of the origin of pollution causing the defect was impossible by visual inspection. Therefore, samples were collected from the inside of the system and comparison of the microbial biota in the slime with that in the defect portion was performed. The samples were collected from:

　　1) slime in the head box,
　　2) slime beneath the wheel,
　　3) slime on the wall of wahite water silo,
　　4) slime on the wall of stuff box,
　　5) slime in tubes of CP (consistency profiling control system) and
　　6) white water.

DNA extraction was performed in the procedures as given above for the precipitate obtained by the centrifugation, for the slimes, from each an about 50 mg of wet slime and, for the white water, from 2 ml of white water, by subjecting to centrifugation at 10,000 rpm at 4 °C for 10 minutes. The dried centrifugation precipitate was suspended in 50 μl of aqueous TE solution.

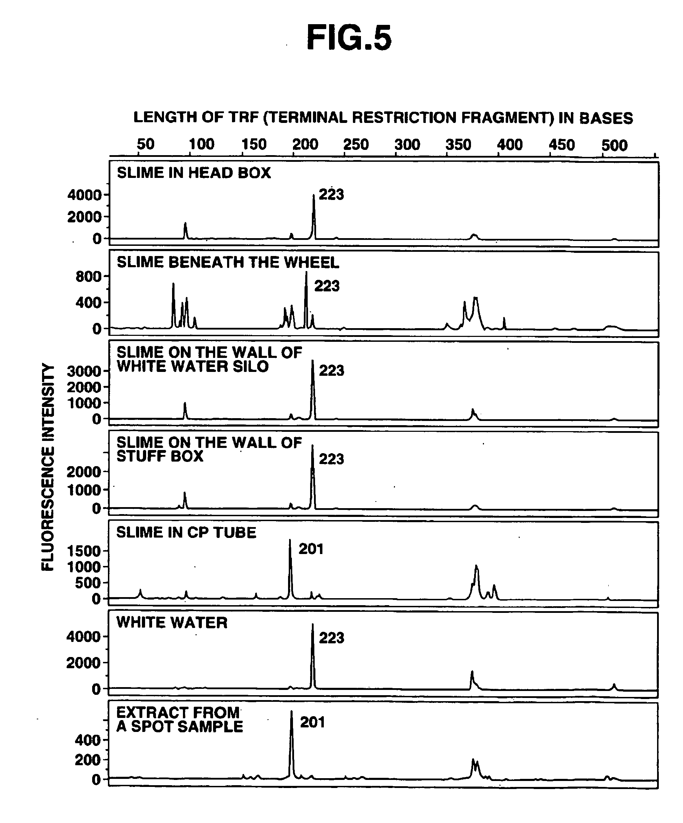

For the comparison of the microbial biota, TRFLP method was employed. PCR was carried out under the same condition as given above using, as the template, the DNA suspensions from the slime and from the white water as well as the DNA suspension from the defect. For the primer, one which is labelled at the 5'-terminal of Bact27f with 4,4,2', 4',5',7'-hexachloro-6-carboxylfluorescein (6-HEX) was used. 25 μl of aqueous solution of amplified 16S rDNA were passed to MicroSpin S-400 HR column (Amersham Pharmacia Biotech Corp.) to remove the unreacted primer, whereupon 0.5 μl thereof were mixed with 2 μl of NE2 buffer solution containing 2 units of the restriction enzyme BstUI (of New England Biolabo Corp.) to effect the reaction at 65 °C for 2 hours. To the reaction mixture, there were added 12 μl of formamide and 0.5 μl of Gene-Scan 500 Rox Size Standard (Applied Biosystems Japan, Ltd.) succeedingly and the mixture was agitated sufficiently. The reaction of the mixture was effected at 94°C for two minutes, whereupon the reaction mixture was cooled promptly in ice water and, then, it was subjected to fragment analysis using ABI PRISM 310 Genetic Analyzer in accordance with the GeneScan Program (Applied Biosystems Japan, Ltd.). The results of analysis are shown in Fig. 5.

One bacterium exhibits an intrinsic peak pattern except by, for example, the polymorphism due to content of a plurality of copies, the overlapping of the base sequence for discriminating the restriction enzyme and so on. If the fragment patterns as to the parameter constituted of the peak position and the amplitude are similar between two samples to be compared, it is judged that the microbial biota is nearly similar between the two. As seen from Fig. 5, the fragment pattern for the sample from the defect is mainly based on TRF (terminal restriction fragment) of 201 bases, which resembles the pattern for the sample from CP tube slime but is different clearly from the patterns for those of the white water mainly of the TRF of 223 bases and of others.

From the above analysis results, it was assumed that the defect was caused by introduction of the slime formed in CP tube into the papermaking process course upon slough off or the like. In the CP line, clear white water obtained by treating white water through a polydiscfilter was used, which had hitherto not been subjected to a slime control treatment. Therefore, treatment was now applied by intermittent introduction of a slime control agent containing as the main component 2,2-dibromo-3-nitrilopropionamide into the clear white water pit twice a day. Occurrence of slime formation in the CP tube became thenceforth decreased remarkably and occurrence of defect was also removed.

INDUSTRIAL APPLICABILITY

The method for selecting antimicrobial agent can be utilized, for example, in antimicrobial teratments, in monitoring antimicrobial effect, in slime control in paper manufacturing process and in the selection of antimicrobial agent (slime control agent) for microbe inhibition in, for example, paper manufacturing process, since it permits to select an optimum antimicrobial agent simply within a brief time.

The process for effecting antimicrobial treatment can afford to effect an efficient antimicrobial treatment by permitting selection of an optimum industrial antimicrobial agent in accordance with each specific microbial biota of the objective system within a brief time.

The method for monitoring antimicrobial treatment effect can afford to grasp within a brief time in a simple and assured manner whether the effect of the antimicrobial agent is revealed or not, whereby troubles caused by growth of microbes can be prevented beforehand.

The method for inhibiting slime formation can afford to effect inhibition of slime formation efficiently with least requisite amount of slime control agent, due to permission of assured selection of a slime control agent adapted at the most to each resistant microbe within a brief time.

The method for analysing cling substance on the product of paper manufacturing process can afford to identify the site of origin of occurrence of the causal microbe that caused the defect on the paper product in a simple and assured manner.

The method for inspecting the causal basis of occurrence of cling substance on products in paper manufacturing process can afford to detect the location of occurrence of the causal microbe that caused the defect on the paper prodect in a simple and assured manner.

The method for controlling microbes in paper manufacturing process courses can afford to determine the site of origin of occurrence of the causal microbe that caused the defect on paper product in a simple and assured manner to thereby enable, based on it, to reduce occurrence of defect on paper product in a simple and assured manner.

SEQUENCE LISTING

<110> KURITA WATER INDUSTRIES LTD.

<120> Method for selecting antimicrobial agents and method for utilizing thereof

<130> KWI00-095

<150> JP 2001-1427
<151> 2001-1-9

<150> JP 2001-365004
<151> 2001-11-29

<160> 8

<210> 1
<211> 19
<212> DNA
<213> Artificial Sequence

<220>
<222> (1)...(19)
<223> primer

<400> 1
gagtttgatc mtggctcag        19


<210> 2
<211> 21
<212> DNA
<213> Artificial Sequence


<220>
<222> (1)...(21)
<223> primer


<400> 2
acggytacct tgttacgact t        21


<210> 3
<211> 18
<212> DNA
<213> Artificial Sequence


<220>
<222> (1)... (18)
<223> primer


<400> 3
cagcmgccgc ggtaatwc        18


<210> 4
<211> 19
<212> DNA
<213> Artificial Sequence


<220>
<222> (1)... (19)
<223> primer


<400> 4
gtagtcatat gcttgtctc        19


<210> 5
<211> 21
<212> DNA
<213> Artificial Sequence


<220>
<222> (1)... (21)
<223> primer


<400> 5
ggctgctggc accagacttg c        21


<210> 6
<211> 501
<212> DNA
<213> Unknown


<220>
<223> Partial nucleotide sequence of 18S rDNA of filamentous fungi obtai ned from the process of the paper

manufacture.

<400> 6

```
taagccatgc atgtctaagt ataagcaagt atactgtgaa actgcgaatg gctcattaaa    60

tcagttatcg tttatttgat agtgcccttt actacatgga taaccgtggt aattctagag   120

ctaatacatg catcaagccc cgaccagggg tgtatttatt agataaaaaa ccaatgccct   180


tttgggctgt ttggtgagtc atgataacgc aacgaatcgc atggccttgc gccggcgatg   240

gttcattcaa atttctgccc tatcaacttt cgattgtagt ttagtggact acaatggttt   300

caacgggtaa cggggaatta gggttcgact ccggagaggg agcctgagaa acggctacca   360

catccaagga aggcagcagg cgcgcaaatt acccaatccc gacacgggga ggtagtgaca   420

ataaatactg atacagggct cttttgggtc ttgtaattgg aatgagtaca atttaaatcc   480

cttaacgagg aacaattgga g                                              501
```

<210> 7
<211> 20
<212> DNA
<213> Artificial Sequence

<220>
<222> (1)...(20)
<223> primer

<400> 7
agagtttgat cmtggctcag        20

<210> 8
<211> 18
<212> DNA
<213> Artificial Sequence

<220>
<222> (1)... (18)
<223> primer

<400> 8
gwattaccgc ggckgctg        18

**Claims**

1. A method for inspecting the location of the origin of occurrence of the microbes that causes cling substances on products of manufacture in paper manufacturing process, comprising

a microbe analyzing step of the products comprising extracting from the cling substance adhering on the product of manufacture the DNA originated from each microbe, and performing analysis of the microbial biota or the dominant microbe of the cling substance by making use of the resulting base sequence of DNA as analysis parameter,

a microbe analyzing step of the slimes comprising collecting slimes at at least two locations in the paper manufacturing process selected from the group consisting of pulp raw material preparation system, broke system, paper manufacturing chemical preparation system, stock preparation system, white water circulation system and whitewater recovering system, extracting from the collected slimes the DNA originated from each microbe, and performing analysis of the microbial biota or the dominant microbe of the slimes by making use of the resulting base sequence of DNA as analysis parameter, and

a determination step comprising comparing the microbial biota of the dominant microbe in the cling substance with the microbial biota or the dominant microbe in the slimes collected at said at least two locations in the paper manufacturing process courses, identifying the slime that caused the cling substance by judging the slime of which the microbial biota or the dominant microbe that is in accord with or close to that in the cling substance as the causal slime, and determining the location of occurrence of the slime that caused the cling substance by judging the location where the identified slime has been collected as the location of occurrence of the causal slime.

2. A method for controlling microbes that causes cling substances on products in paper manufacturing process courses, comprising

a microbe analyzing step of the products comprising extracting from the cling substance adhering on the product of manufacture the DNA originated from each microbe, and performing analysis of the microbial biota or the dominant microbe of the cling substance by making use of the resulting base sequence of DNA as analysis parameter,

a microbe analyzing step of the slimes comprising collecting slimes at at least two locations in the paper manufacturing process selected from the group consisting of pulp raw material preparation system, broke system, paper manufacturing chemical preparation system, stock preparation system, whitewater circulation system and whitewater recovering system, extracting from the collected slimes the DNA originated from each microbe, and performing analysis of the microbial biota or the dominant microbe of the slimes by making use of the resulting base sequence of DNA as analysis parameter,

a determination step comprising comparing the microbial biota or the dominant microbe in the cling substance with the microbial biota or the dominant microbe in the slimes collected at said at least two locations in the paper manufacturing process courses, identifying the slime that caused the cling substance by judging the slime of which the microbial biota or the dominant microbe that is in accord with or close to that in the cling substance as the causal slime, and determining the location of occurrence of the slime that caused the cling substance by judging the location where the identified slime has been collected as the location of occurrence of the causal slime, and

a controlling step comprising performing an antimicrobial/antiseptic treatment at the so-determined location of occurrence of the slime.

3. The method as claimed in claim 1 or 2, wherein the DNA to be used for analyzing the microbial biota or the dominant microbe is that which codes ribosomal RNA.

**Patentansprüche**

1. Verfahren zum Prüfen des Herkunftsursprungs der Mikroben, die Haftstoffe auf Herstellungserzeugnissen im Papierherstellungsverfahren hervorrufen, umfassend

einen Mikrobe-Analyseschritt der Erzeugnisse, umfassend das Extrahieren der DNA, die von jeder Mikrobe stammt, aus dem Haftstoff, der dem Herstellungserzeugnis anhaftet, und das Durchführen der Analyse der mikrobiellen Biota oder der vorherrschenden Mikrobe des Haftstoffes unter Verwendung der sich ergebenden Basensequenz der DNA als Analyseparameter,

einen Mikrobe-Analyseschritt der Schleime, umfassend das Abnehmen von Schleimen an mindestens zwei Stellen im Papierherstellungsverfahren, ausgewählt aus der Gruppe bestehend aus Pulpe-Rohmaterial-Zubereitungssystem, Kollerstoffsystem, chemisches Papierherstellungs-Zubereitungssystem, Stoffzubereitungssystem, Siebwasser-Umwälzungssystem, Siebwasser-Wiederherstellungssystem; das Extrahieren der DNA, die aus jeder Mikrobe stammt, von den abgenommenen Schleimen, sowie das Durchführen der Analyse der mikrobiellen Biota oder der vorherrschenden Mikrobe der Schleime unter Verwendung der sich ergebenden Basensequenz der DNA als Analyseparameter, sowie

einen Bestimmungsschritt, umfassend das Vergleichen der mikrobiellen Biota der vorherrschenden Mikrobe in dem Haftstoff mit den mikrobiellen Biota oder der vorherrschenden Mikrobe in den Schleimen, die an mindestens zwei Stellen in den Papierherstellungsverfahrensverläufen abgenommen wurden, wobei der Schleim, der den Haftstoff verursacht hat, identifiziert wird durch das Beurteilen des Schleimes, dessen mikrobielle Biota oder vorherrschende Mikrobe in Übereinstimmung mit oder nahe derjenigen im Haftstoff ist als den verursachenden Schleim, und Bestimmen der Auftretensstelle des Schleimes, der den Haftstoff verursacht hat durch das Beurteilen der Stelle, an der der identifizierte Schleim abgenommen wurde als die Auftretensstelle des verursachenden Schleimes.

**2.** Verfahren zum Kontrollieren von Mikroben, die Haftstoffe auf Erzeugnissen im Papierherstellungsverfahrensverlauf verursachen, umfassend

einen Mikrobe-Analyseschritt der Erzeugnisse, umfassend das Extrahieren der DNA, die von jeder Mikrobe stammt, aus dem Haftstoff, der dem Herstellungserzeugnis anhaftet, und das Durchführen der Analyse der mikrobiellen Biota oder der vorherrschenden Mikrobe des Haftstoffes unter Verwendung der sich ergebenden Basensequenz der DNA als Analyseparameter,

einen Mikrobe-Analyseschritt der Schleime, umfassend das Abnehmen von Schleimen an mindestens zwei Stellen im Papierherstellungsverfahren, ausgewählt aus der Gruppe bestehend aus Pulpe-Rohmaterial-Zubereitungssystem, Kollerstoffsystem, chemisches Papierherstellungs-Zubereitungssystem, Stoffzubereitungssystem, Siebwasser-Umwälzungssystem, Siebwasser-Wiederherstellungssystem; das Extrahieren der DNA, die aus jeder Mikrobe stammt, von den abgenommenen Schleimen, sowie das Durchführen der Analyse der mikrobiellen Biota oder der vorherrschenden Mikrobe der Schleime unter Verwendung der sich ergebenden Basensequenz der DNA als Analyseparameter,

einen Bestimmungsschritt, umfassend das Vergleichen der mikrobiellen Biota der vorherrschenden Mikrobe in dem Haftstoff mit den mikrobiellen Biota oder der vorherrschenden Mikrobe in den Schleimen, die an mindestens zwei Stellen in den Papierherstellungsverfahrensverläufen abgenommen wurden, wobei der Schleim, der den Haftstoff verursacht hat, identifiziert wird durch das Beurteilen des Schleimes, dessen mikrobielle Biota oder vorherrchende Mikrobe in Übereinstimmung mit oder nahe derjenigen im Haftstoff ist als den verursachenden Schleim, und Bestimmen der Auftretensstelle des Schleimes, der den Haftstoff verursacht hat durch das Beurteilen der Stelle, an der der identifizierte Schleim abgenommen wurde als die Auftretensstelle des verursachenden Schleimes, und einen Kontrollschicht, umfassend das Durchführen einer anti-mikrobiellen/anti-septischen Behandlung an der so bestimmten Auftretensstelle des Schleimes.

**3.** Verfahren wie in Anspruch 1 oder 2 beansprucht, wobei die DNA, die für das Analysieren der mikrobiellen Biota oder der vorherrschenden Mikrobe verwendet werden soll, diejenige ist, die für ribosomale RNA kodiert.

**Revendications**

**1.** Procédé pour inspecter l'emplacement de l'origine de survenance des microbes qui amènent des substances adhérentes sur des produits de fabrication dans un processus de fabrication de papier, comprenant

une étape d'analyse de microbe des produits comprenant l'extraction depuis la substance adhérente adhérant sur le produit de fabrication de l'ADN originaire de chaque microbe, et l'exécution d'une analyse du biote microbien ou du microbe dominant de la substance adhérente en utilisant la séquence de base résultante d'ADN en tant que paramètre d'analyse,

une étape d'analyse de microbe des boues comprenant la collecte de boues au niveau d'au moins deux emplacements dans le processus de fabrication de papier sélectionné à partir du groupe constitué par un système de préparation de matière première pulpeuse, un système de rebuts, un système de préparation chimique de fabrication de papier, un système de préparation de pâte, un système de circulation d'eau blanche et un système de récupération d'eau blanche, l'extraction à partir des boues collectées de l'ADN originaire de chaque microbe, et l'exécution d'une analyse du biote microbien ou du microbe dominant des boues en se servant de la séquence de base résultante d'ADN en tant que paramètre d'analyse, et

une étape de détermination comprenant la comparaison du biote microbien ou du microbe dominant dans la substance adhérente au biote microbien ou au microbe dominant dans les boues collectées au niveau desdits au moins deux emplacements au cours du processus de fabrication de papier, l'identification de la boue qui a amené la substance adhérente en déterminant la boue de celui du biote microbien ou du microbe dominant qui est en accord avec ou proche de celle dans la substance adhérente en tant que boue causale, et la détermination de l'emplacement de survenance de la boue qui a amené la substance adhérente en déterminant l'emplacement où la boue identifiée a été collectée en tant qu'emplacement de survenance de la boue causale.

**2.** Procédé pour maîtriser des microbes qui amènent des substances adhérentes sur des produits au cours d'un processus de fabrication de papier, comprenant

une étape d'analyse de microbe des produits comprenant l'extraction à partir de la substance adhérente adhérant sur le produit de fabrication de l'ADN originaire de chaque microbe, et l'exécution d'une analyse du biote microbien ou du microbe dominant de la substance adhérente en utilisant la séquence de base résultante d'ADN en tant que paramètre d'analyse,

une étape d'analyse de microbe des boues comprenant la collecte de boues au niveau d'au moins deux emplacements dans le processus de fabrication de papier sélectionné à partir du groupe constitué par un système de préparation de matière première pulpeuse, d'un système de rebuts, d'un système de préparation chimique de fabrication de papier, d'un système de préparation de pâte, d'un système de circulation d'eau blanche et d'un système de récupération d'eau blanche, l'extraction à partir des boues collectées de l'ADN originaire de chaque microbe, et l'exécution d'une analyse du biote microbien ou du microbe dominant des boues en utilisant la séquence de base résultante d'ADN en tant que paramètre d'analyse,

une étape de détermination comprenant la comparaison du biote microbien ou du microbe dominant dans la substance adhérente au biote microbien ou au microbe dominant dans les boues collectées au niveau desdits au moins deux emplacements au cours du processus de fabrication de papier, l'identification de la boue qui a amené la substance adhérente en déterminant la boue de celui du biote microbien ou du microbe dominant qui est en accord avec ou proche de celle dans la substance adhérente en tant que boue causale, et la détermination de l'emplacement de survenance de la boue qui a amené la substance adhérente en déterminant l'emplacement où la boue identifiée a été collectée en tant qu'emplacement de survenance de la boue causale, et

une étape de commande comprenant l'exécution d'un traitement antimicrobien / antiseptique au niveau de l'emplacement ainsi déterminé de survenance de la boue.

3. Procédé selon la revendication 1 ou 2, dans lequel l'ADN devant être utilisé pour analyser le biote microbien ou le microbe dominant est celui qui code de l'ARN ribosomique.

# FIG.1

TREATMENT RESULT

6 — CONTROL SYSTEM

5 — ANTIMICROBIAL AGENT SELECTING SYSTEM

4 — MICROBIAL BIOTA ANALYZING SYSTEM

28a, 27a

7a

27b

7b

28b

27c, 28c

7c

ANTIMICROBIAL AGENT STORAGE

2 STUFF BOX

1 PAPER MACHINE

18, 17, 21, 22, 23

15, 24

14

12

11

13

PULP SLURRY

3 WHITE WATER PIT

EP 1 350 431 B1

# FIG.2

START

SAMPLING — 31

DETERMINATION OF NUMBER OF COLONIES AND TOTAL NUMBER OF MICROBES — 32

33
WERE NUMBER OF COLONIES ≒TOTAL NUMBER OF MICROBES? — NO

YES 34
rDNA ANALYSIS OF COLONY-FORMING MICROBE

35
rDNA ANALYSIS OF ALL MICROBES

SEARCH AT DATA BASE OF MICROBES — 36

IDENTIFICATION OF THE MICROBE — 37

SEARCH AT DATA BASE OF ANTIMICROBIAL AGENTS — 38

39
WAS AN ANTIMICROBIAL AGENT PICKED UP ? — NO

42
DOES THE IDENTIFIED MICROBE FORM A COLONY? — YES

43
CULTURE EXPERIMENT FOR EACH ANTIMICROBIAL AGENT

NO

44
SELECTION OF ANTIMICROBIAL AGENT

YES

40
IS THERE ANY SUCCESSFUL EXAMPLE IN SYSTEM OF SIMILAR ENVIRONMENTAL CONDITION ? — NO

45
SELECTION OF ANTIMICROBIAL AGENT BASED ON EXPERIENCE

YES 41
SELECTION OF ANTIMICROBIAL AGENT

IMPLEMENTATION OF ANTIMICROBIAL TREATMENT — 46

47
WAS THE EFFECT OF TREATMENT BETTER? — YES

NO 48
THE RESULTS ARE INPUTTED INTO THE DATA BASE OF ANTIMICROBIAL AGENT

49
THE RESULTS ARE INPUTTED INTO THE DATA BASE OF ANTIMICROBIAL AGENT

50
INSERT A VOLUNTARY PERIOD OF TIME

MICROBIOTA ANALYZING SYSTEM

ANTIMICROBIAL AGENT SELECTING SYSTEM

APPLICATION

ANTIMICROBIAL AGENT SELECTING SYSTEM

40

# FIG.3

# FIG.4

DIRECTION OF
PHORESIS

LANE 1 : MOLECULAR WEIGHT MARKER Φ×174 / HincII digest

LANES 2, 3 : NORMAL PART EXTRUDED DNA,
WITH BACTERIOSPECIFIC PRIMER

LANES 4, 5 : DEFECTIVE PART EXTRUDED DNA,
WITH BACTERIOSPECIFIC PRIMER

LANES 6, 7 : NORMAL PART EXTRUDED DNA,
WITH FUNGALSPECIFIC PRIMER

LANES 8, 9 : DEFECTIVE PART EXTRUDED DNA,
WITH FUNGALSPECIFIC PRIMER

# FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001001427 A

- JP 2001365004 A

### Non-patent literature cited in the description

- **Vaisanen et al.** *Journal of Appl. Bacteriology,* 1991, vol. 71, 130-133
- **Moyer et al.** *Applied and Environmental Microbiology,* 1996, vol. 62, 2501-2507
- **Muyzer et al.** Denatured Gradient Gel Electrophoresis. *Applied and Environmental Microbiology,* 1993, vol. 59, 695-700
- **Eichner et al.** Temperature Gradient Gel Electrophoresis. *Applied and Environmental Microbiology,* 1999, vol. 65, 102-109
- **Schwieger et al.** Single Strand Conformational Polymorphism. *Applied and Environmental Microbiology,* 1998, vol. 64, 4870-4876
- **Liu et al.** Terminal Restiction Fragment Length Polymorphism. *Applied and Environmental Microbiology,* 1997, vol. 63, 4516-4522

- **Dunbar et al.** *Applied and Environmental Microbiology,* 1999, vol. 65, 1662-1669
- **Wittwer et al.** *BioTechnique,* 1997, vol. 22, 130-138
- **Amman et al.** Fluorescence In Situ Hybridization. *Applied and Environmental Microbiology,* 1992, vol. 58, 614-623
- **William et al.** *Microbiology,* 1995, vol. 141, 2793-2800
- **Muyzer et al.** *Applied and Environmental Microbiology,* 1993, vol. 59, 695-700
- **Eichner et al.** *Applied and Environmental Microbiology,* 1999, vol. 65, 102-109
- **Schwieger et al.** *Applied and Environmental Microbiology,* 1998, vol. 64, 4870-4876
- **Liu et al.** *Applied and Environmental Microbiology,* 1997, vol. 63, 4516-4522